# DEMANDE DE BREVET EUROPEEN

(11) **EP 2 347 652 A1**
(43) Date de publication de la demande: **27.07.2011**
(21) Numéro de dépôt: 10190722.8
(22) Date de dépôt: 23.01.2009
(51) Int. Cl.: A01N 25/02, C07C 231/02, C07C 235/74, C08K 5/20, C09D 7/12, C11D 7/50, A01N 33/22, A01N 47/24, A01N 57/12, A01N 37/26, A01N 43/653, A01N 47/22, A01N 53/00, A01N 57/16

(54) **Esteramides, leur procédé de préparation et leur utilisation**

(30) Priorité: 25.01.2008 FR 0800393; 18.09.2008 FR 0805133
(62) Demande divisionnaire de: 09704290.7
(71) Demandeur: Rhodia Opérations, 93306 Aubervilliers (FR)
(72) Inventeur: Jentzer, Olivier, 69390 Vourles (FR); Guglieri, Massimo, 75005 Paris (FR)
(74) Mandataire: Blot, Philippe Robert Emile

(57) **Abrégé**

La présente invention a pour objet l'utilisation comme solvants de composés de type esteramides. Elle a également pour objet des procédés particulièrement pratiques pour la préparation d'esteramides. Elle a également pour objet des esteramides, pouvant notamment être utilisés comme solvants, par exemple dans des formulations phytosanitaires.

## Description

La présente invention a pour objet l'utilisation comme solvants de composés de type esteramides. Elle a également pour objet des procédés particulièrement pratiques pour la préparation d'esteramides. Elle a également pour objet de nouveaux esteramides, pouvant notamment être utilisés comme solvants, par exemple dans des formulations phytosanitaires.

L'industrie utilise de nombreux composés chimiques à titre de solvants, par exemple pour préparer des produits chimiques et des matériaux, pour formuler des composés chimiques, ou pour traiter des surfaces. Par exemple des solvants sont utilisés pour la formulation d'actifs phytosanitaires notamment sous forme de concentrés émulsionnables (Emulsifiable Concentrate "EC") destinés à être dilués dans de l'eau par l'exploitant agricole, avant application sur un champ.

L'industrie est à la recherche de nouveaux composés permettant de varier ou d'optimiser les produits et procédés dans lesquels des solvants, notamment des solvants polaires, sont à utiliser. L'industrie a notamment besoin de composés de coût modeste, présentant des propriétés d'usage intéressantes. L'industrie a également besoin de composés présentant un profile toxicologique et/ou écologique perçu comme favorable, notamment une faible volatilité (faible VOC), une bonne biodégradabilité, une faible toxicité et/ou une faible dangerosité.

On connaît l'utilisation comme solvants des dialkylamides. Il s'agit de produits de formule R-CONMe₂ ou R est un groupe hydrocarbonné comme un alkyle, typiquement en C₆-C₃₀. De tels produits sont notamment commercialisés sous la dénomination Genagen® par la société Clariant. Ces solvants trouvent des applications notamment dans le domaine phytosanitaire.

On connaît également comme solvants les diesters d'acides dicarboxyliques, notamment les diesters obtenus par estérification d'un mélange d'acide adipique, d'acide glutarique et d'acide succinique. De tels produits sont notamment commercialisés sous les dénominations Rhodiasolv® RPDE et Rhodiasolv® DIB par la société Rhodia.

Le document US 4588833 (dont les demandes prioritaires ont été publiées comme DE 3339386 et DE 3420112) décrit un procédé de préparation d'esteramides par réaction à haute température, catalysée par le cobalt, d'un amide insaturé avec un alcool et du monoxyde de carbone. Il est par ailleurs mentionné que les composés préparés peuvent être utilisés comme stabilisants de polymères. Les esteramides préparés sont les suivants :

| | |
|---|---|
| Exemple 1 | Mélange de A et B |
| | A: PhOOC-CH(CH₃)-CH₂-CONEt₂ |
| | B: PhOOC-CH₂-CH₂-CH₂-CONEt₂ |
| Exemples 2-6 | C: EtOOC-CH(CH₃)-CH₂-CONEt₂ |
| Exemple 7 | D: MeOOC-CH(CH₃)-CH₂-CONEt₂ |
| Exemples 8-9 | E: Me-CH(OMe)-OOC-CH(CH₃)-CH₂-CONEt₂ |
| Exemple 10 | F: Cyclohexyl-OOC-CH(CH₃)-CH₂-CONEt₂ |
| Exemple 11 | G: Ph-CH₂OOC-CH(CH₃)-CH₂-CONEt₂ (de par les réactifs) |
| Exemple 12 | H: p-cresyl―OOC-CH(CH₃)-CH₂-CONEt₂ |
| Exemple 18-20 | I: EtOOC-CHEt-CH₂-CONEt₂ |
| | + J: EtOOC-CH(CH₃)-CH₂-CH₂-CONEt₂ |
| | + K: EtOOC-CH₂-CH₂-CH₂-CH₂-CONEt₂ |

Le composé de formule L: MeOOC-CHEt-CH₂-CONMe₂ a été identifié par le CAS Registry Number® 368212-04-8, en référence avec le document WO 01/79167 portant sur un domaine éloigné, et dont la pertinence est douteuse.

Le composé de formule M: MeOOC-CH₂-CH(CH₃)-CH₂-CONH(n-butyle) a été identifié par le CAS Registry Number® 538326-02-2, en référence avec un document portant sur des réactions enzymatiques.

Le composé de formule N: Me00C-CH₂-CH(CH₃)-CH₂-CONMe₂ a été identifié par le CAS Registry Number® 70367-41-8, en référence avec un document portant sur la mise en oeuvre d'énolates de lithium.

Le document US 3417114 décrit dans l'exemple 9 le composé désigné par DMGME, de formule O: MeOOC-CH₂-CH₂-CH₂-CONMe₂- Ce composé est préparé par réaction de glutarate de diméthyle avec de la diméthylamine, puis isolation du DMGME par distillation à partir du mélange complexe obtenu (il s'agit en fait d'un sous produit). Le DMGME est dit présenter un point de fusion de 7.5°C.

Le document US 3288794 comprend des enseignements similaires.

Le document US 4020099, mentionne des produits comme le DMGME et mentionne par ailleurs la cristallisation du diphenyle téréphtalate dans des solvants. Le DMGME n'est toutefois pas mis en oeuvre.

Le composé de formule P: MeOOC-CH₂-CH₂-CONMe₂ a été identifié par le CAS Registry Number® 30891-34-0, en référence avec des documents portant sur des domaines éloignés, et dont la pertinence est douteuse.

Le document DE 1040234 décrit les composés suivants et leur utilisation comme plastifiants :
C₄Hg-OOC-CH₂-CH₂-CONEt₂
C₆H₁₃-OOC-(CH₂)₈-CON(C₃H₇)₂
C₈H₁₇-OOC-(CH₂)₈-CON(C₄H₉)₂
C₈H₁₇-OOC-(CH₂)₈-CON(C₈H₁₇)₂

II demeure un besoin, comme expliqué plus haut, pour de nouveaux solvants, notamment dans les formulations phytosanitaires, et pour de nouveaux composés. Il existe également un besoin pour des procédés de préparation d'esteramides plus efficaces.

L'invention répond à au moins un des besoins mentionnés ci-dessus en proposant l'utilisation comme solvant ou agent de coalescence, d'un composé esteramide de formule (I) suivante:

R¹OOC-A-CONR²R³ (I)

où:
- R¹ est un groupe choisi parmi les groupes hydrocarbonés comprenant un nombre moyen d'atomes de carbone allant de 1 à 36, saturés ou insaturés, linéaires ou ramifiés, éventuellement cycliques, éventuellement aromatiques,
- R² et R³, identiques ou différents, sont des groupes choisis parmi les groupes hydrocarbonés comprenant un nombre moyen d'atome de carbone allant de 1 à 36, saturés ou insaturés, linéaires ou ramifiés, éventuellement cycliques, éventuellement aromatiques, éventuellement substitués, R² et R³pouvant éventuellement former ensemble un cycle, éventuellement substitué et/ou comprenant éventuellement un hétéroatome, et
- A est un groupe alkyle divalent linéaire ou ramifié comprenant un nombre moyen d'atomes de carbone allant de 2 à 12, de préférence de 2 à 4.

On mentionne que R¹ peut être un groupe différent d'un groupe menthyle.

L'invention a également pour objet un procédé de solvatation, co-solvatation, plastification, coalescence et/ou inhibition de cristallisation par ajout du composé de l'invention. L'invention a également pour objet des formulations comprenant le composé de l'invention. Les formulations peuvent être notamment des formulations phytosanitaires.

Selon un mode particulier de l'invention, si A est linéaire, alors le composé esteramide est utilisé dans une composition phytosanitaire, une composition de nettoyage, de dégraissage ou de décapage. Selon un autre mode particulier, si A est ramifié, alors le composé esteramide est utilisé dans une composition phytosanitaire, une composition de nettoyage, de dégraissage ou de décapage.

Selon un mode particulier de l'invention, si R² et R³ sont des groupes éthyles, alors le composé esteramide est utilisé dans une composition phytosanitaire, une composition de nettoyage, de dégraissage ou de décapage.

L'invention a également pour objet au moins un procédé de préparation du composé esteramide.

L'invention a également pour objet de nouveaux composés esteramides pouvant être particulièrement adaptés aux utilisations mentionnées ci-dessus. A ce titre l'invention concerne également un composé esteramide de formule (1) suivante :

R¹OOC-A-CONR²R³ (I)

où:
- R¹ est un groupe choisi parmi les groupes hydrocarbonés comprenant un nombre moyen d'atomes de carbone allant de 1 à 36, saturés ou insaturés, linéaires ou ramifiés, éventuellement cycliques, éventuellement aromatiques,
- R² et R³, identiques ou différents, sont des groupes choisis parmi les groupes hydrocarbonés comprenant un nombre moyen d'atomes de carbone allant de 1 à 36, saturés ou insaturés, linéaires ou ramifiés, éventuellement cycliques, éventuellement aromatiques, éventuellement substitués, R² et R³ pouvant éventuellement former ensemble un cycle, éventuellement substitué et/ou comprenant éventuellement un hétéroatome, et
- A est un groupe alkyle divalent linéaire ou ramifié comprenant un nombre moyen d'atomes de carbone allant de 2 à 12, de préférence de 2 à 4, à l'exception des composés ou mélanges suivants:
   - MeOOC-CHEt-CH₂-CONMe₂
   - MeOOC-CH₂-CH(CH₃)-CH₂-CONMe₂
   - MeOOC-CH₂-CH₂-CH₂-CONMe₂
   - MeOOC-CH₂-CH₂-CONMe₂
   - mélange de PhOOC-CH(CH₃)-CH₂-CONEt₂ et PhOOC-CH₂-CH₂-CH₂-CONEt₂
   - EtOOC-CH(CH₃)-CH₂-CONEt₂
   - MeOOC-CH(CH₃)-CH₂-CONEt₂
   - Me-CH(OMe)-OOC-CH(CH₃)-CH₂-CONEt₂
   - Cyclohexyl-OOC-CH(CH₃)-CH₂-CONEt₂
   - Ph-CH₂OOC-CH(CH₃)-CH₂-CONEt₂
   - p-cresyl―OOC-CH(CH₃)-CH₂-CONEt₂
   - mélange de EtOOC-CHEt-CH₂-CONEt₂, EtOOC-CH(CH₃)-CH₂-CH₂-CONEt₂ et EtOOC-CH₂-CH₂-CH₂-CH₂-CONEt₂,
   - MeOOC-CH₂-CH(CH₃)-CH₂-CONH(n-butyle),
   si ces derniers, individuellement, ne sont pas utilisés en mélange avec d'autres composés répondant à la formule (I).

On peut également exclure les composés ou mélanges suivants:
C₄Hg-OOC-CH₂-CH₂-CONEt₂
C₆H₁₃-OOC-(CH₂)₈-CON(C₃H₇)₂
C₈H₁₇-OOC-(CH₂)₈-CON(C₄H₉)₂
C₈H₁₇-OOC-(CH₂)₈-CON(C₈H₁₇)₂
si ces derniers, individuellement, ne sont pas utilisés en mélange avec d'autres composés répondant à la formule (I).

### Définitions

Dans la présente demande le terme solvant est entendu dans un sens large, couvrant notamment les fonctions de co-solvant, d'inhibiteur de cristallisation, de décapant. Le terme solvant peut notamment désigner un produit liquide à la température d'utilisation, de préférence de point de fusion inférieur ou égal à 20°C, de préférence à 5°C, de préférence à 0°C, pouvant contribuer à rendre liquide une matière solide, ou à empêcher ou retarder la solidification ou la cristallisation de matière dans un milieu liquide.

Dans la présente demande un "composé de l'invention" désigne tout composé répondant à la formule générale (I). On mentionne que le terme "composé" couvre également des mélanges de plusieurs molécules répondant à le formule générale (I). Il peut donc s'agir d'une molécule de formule (I) ou d'un mélange de plusieurs molécules de formule (I). Un tel composé peut notamment être un "composé de l'invention nouveau" excluant les composés ou mélanges suivants (ces derniers pouvant être désignés comme "composés de l'invention anciens") :
- MeOOC-CHEt-CH₂-CONMe₂
- MeOOC-CH₂-CH(CH₃)-CH₂-CONMe₂
- MeOOC-CH₂-CH₂-CH₂-CONMe₂
- MeOOC-CH₂-CH₂-CONMe₂
- mélange de PhOOC-CH(CH₃)-CH₂-CONEt₂ et PhOOC-CH₂-CH₂-CH₂-CONEt₂
- EtOOC-CH(CH₃)-CH₂-CONEt₂
- MeOOC-CH(CH₃)-CH₂-CONEt₂
-Me-CH(OMe)-OOC-CH(CH₃)-CH₂-CONEt₂
-Cyclohexyl-OOC-CH(CH₃)-CH₂-CONEt₂
- Ph-CH₂OOC-CH(CH₃)-CH₂-CONEt₂
- p-cresyl―OOC-CH(CH₃)-CH₂-CONEt₂
- mélange de EtOOC-CHEt-CH₂-CONEt₂, EtOOC-CH(CH₃)-CH₂-CH₂-CONEt₂ et EtOOC-CH₂-CH₂-CH₂-CH₂-CONEt₂,
- MeOOC-CH₂-CH(CH₃)-CH₂-CONH(n-butyle).

On peut également exclure du champ des "composés de l'invention nouveaux" les composés ou mélanges suivants:
C₄H₉-OOC-CH₂-CH₂-CONEt₂
C₆H₁₃-OOC-(CH₂)₈-CON(C₃H₇)₂
C₈H₁₇-OOC-(CH₂)₈-CON(C₄H₉)₂
C₈H₁₇-OOC-(CH₂)₈-CON(C₈H₁₇)_{2.}

Dans la présente demande, par "composition de matière", on entend une composition, plus ou moins complexe, comprenant plusieurs composés chimiques. Il peut s'agir typiquement d'un produit de réaction non purifié ou modestement purifié. Le composé de l'invention pourra notamment être isolé et/ou commercialisé et/ou utilisé sous forme d'une composition de matière le comprenant. Si le composé de l'invention est un mélange de plusieurs composés de formule (I) alors c'est aussi une composition de matière. Le composé de l'invention, sous forme de molécule pure ou sous forme d'un mélange répondant à la formule (I), peut être compris dans une composition de matière.

Dans la composition de matière le composé de l'invention peut représenter au moins 10% en poids. De préférence, il s'agit du composé principal de la composition de matière. Par composé principal, on entend dans la présente demande, le composé dont la teneur est la plus élevée, même si sa teneur est inférieure à 50% en poids (par exemple dans un mélange de 40% de A, de 30% de B, et de 30% de C, le produit A est le composé principal). Encore plus préférablement le composé de l'invention représente au moins 50% en poids de la composition de matière, par exemple de 70 à 95% en poids, et même de 75 à 90% en poids. Comme indiqué plus haut, la composition de matière peut être un produit de réaction.

### Composé de l'invention

Le composé de l'invention est un composé de formule générale (I) donnée ci-dessus.

Les groupes R¹ R² et R³, identiques ou différents, peuvent notamment être des groupes choisis parmi les groupes alkyle, aryle, alkaryle, arylalkyle en C₁-C₁₂ ou le groupe phényle. Les groupes R² et R³ peuvent éventuellement être substitués, notamment par des groupes hydroxyle.

Le groupe R¹ peut notamment être choisi parmi les groupes méthyle, éthyle, propyle, isopropyle, n-butyle, isobutyle, n-pentyl, isopentyle, isoamyle, n-hexyle, cyclohexyle, 2-éthylbutyle, n-octyle, isooctyle, 2-ethylhexyle, tridecyle.

Les groupes R² et R³, identiques ou différents, peuvent notamment être choisis parmi les groupes méthyle, éthyle, propyle (n-propyl), isopropyle, n-butyle, isobutyle, n-pentyle, amyle, isoamyle, hexyle, cyclohexyle, hydroxyéthyle. Les groupes R² et R³ peuvent également être tels qu'ils forment ensemble avec l'atome d'azote un groupe morpholine, piperazine ou piperidine. Selon des modes particuliers de réalisation, R²=R³= méthyle, ou R²=R³= éthyle, ou R²=R³= hydroxyéthyle.

Selon un mode de réalisation particulier si A comprend un groupe de formule -CH₂-CH₂- et/ou de formule -CH₂-CH₂-CH₂-CH₂- et/ou de formule -(CH₂)₈- linéaire alors il s'agit d'un mélange de groupes A. Selon un mode de réalisation particulier, si A est linéaire, alors il s'agit d'un mélange de groupes A, par exemple d'un mélange de deux ou trois des groupes -CH₂-CH₂- (éthylène); -CH₂-CH₂-CH₂-(n-propylène); et -CH₂-CH₂-CH₂-CH₂- (n-butylène).

Selon un premier mode de réalisation particulier de l'invention le groupe A est un groupe alkyle linéaire divalent choisi parmi les groupes de formules suivantes:
-CH₂-CH₂- (éthylène); -CH₂-CH₂-CH₂- (n-propylène); -CH₂-CH₂-CH₂-CH₂- (n-butylène), et leurs mélanges.

Selon une variante particulière dans ce premier mode de réalisation, le composé de l'invention est choisi parmi les composés suivants:
- MeOOC-CH₂-CH₂-CONMe₂
- MeOOC-CH₂-CH₂-CH₂-CONMe₂
- MeOOC-CH₂-CH₂-CH₂-CONMe₂, en mélange avec MeOOC-CH₂-CH₂-CH₂-CH₂-CONMe₂ et/ou avec MeOOC-CH₂-CH₂-CONMe₂.

Selon un deuxième mode de réalisation particulier de l'invention le groupe A est un groupe alkylène ramifié divalent de l'une des formules (lla), (llb), (llc), (llla), et (lllb) suivantes, ou un mélange d'au moins deux groupes choisis parmi les groupes de formules (lla), (llb), et (llc) ou parmi les groupes de formules (llla) et (lllb), ou un mélange d'au moins deux groupes choisis pour l'un parmi les groupes de formules (lla), (llb), et (llc) et pour l'autres parmi les groupes de formules (llla) et (lllb) :
- (CHR⁷)_{y}-(CHR⁶)ₓ-(CHR⁷)_{z}-CH₂-CH₂- (lla)
- CH₂-CH₂-(CHR⁷)_{z}-(CHR⁶)ₓ-(CHR⁷)y- (llb)
- (CHR⁷)_{z}-CH₂-(CHR⁶)ₓ-CH₂-(CHR⁷)_{y}- (llc)
- (CHR⁷)_{y}-(CHR⁶)ₓ-(CHR⁷)_{z}-CH₂- (llla)
- CH₂-(CHR⁷)_{z}-(CHR⁶)ₓ-(CHR⁷)_{y}- (lllb)
   où:
   - x est un nombre entier supérieur à 0,
   - y est un nombre entier moyen supérieur ou égal à 0,
   - z est un nombre entier moyen supérieur ou égal à 0,
   - R⁶, identique ou différent, est un groupe alkyle en C₁-C₆ de préférence en C₁-C₄, et
   - R⁷, identique ou différent, est un atome d'hydrogène ou un groupe alkyle en C₁-C₆ de préférence en C₁-C₄.

Dans ce deuxième mode particulier le groupe A est de préférence un groupe tel que y = z = 0.

De préférence, dans la formule (lla) et/ou dans la formule (llb):
- x= 1; y = z = 0; R⁶ = méthyle.

De préférence, dans la formule (llla) et/ou dans la formule (lllb):
- x= 1; y = z = 0; R⁶ = éthyle.

Selon une variante particulière dans du deuxième mode de réalisation particulier, le composé de l'invention est choisi parmi les composés suivants, et leurs mélanges :
- MeOOC-A_{MG}-CONMe₂
- MeOOC-A_{ES}-CONMe₂
- PeOOC-A_{MG}-CONMe₂
- PeOOC-A_{ES}-CONMe₂
- CycloOOC-A_{MG}-CONMe₂,
- CycloOOC-A_{ES}-CONMe₂
- EhOOC-A_{MG}-CONMe₂
- EhOOC-A_{ES}-CONMe₂
- PeOOC-A_{MG}-CONEt₂
- PeOOC-A_{ES}-CONEt₂
- CycloOOC-A_{MG}-CONEt₂
- CycloOC-A_{ES}-CONEt₂
- BuOOC-A_{MG}-CONEt₂
- BuOOC-A_{ES}-CONEt₂,
- BuOOC-A_{MG}-CONMe₂,
- BuOOC-A_{ES}-CONMe₂,
- EtBuOOC-A_{MG}-CONMe₂,
- EtBuOOC-A_{ES}-CONMe₂,
- n-HeOOC-A_{MG}-CONMe₂,
- n-HeOOC-A_{ES}-CONMe₂,
   où
- A_{MG} représente un groupe MGₐ de formule -CH(CH₃)-CH₂-CH₂-, ou MG_{b} de formule
-CH₂-CH₂-CH(CH₃)- ou un mélange de groupes MGₐ et MG_{b}
- A_{ES} représente un groupe ESₐ de formule -CH(C₂H₅)-CH₂-, ou ES_{b} de formule
-CH₂-CH(C₂H₅)- ou un mélange de groupes ESₐ et ES_{b}
- Pe représente un groupe pentyle, de préférence isopentyle ou isoamyle,
- Cyclo représente un groupe cyclohexyle
- Eh représente un groupe 2-éthylhexyle,
- Bu représente un groupe butyle, de préférence n-butyle ou tertiobutyle,
- EtBu représente un groupe éthylbultyle,
- n-He représente un groupe n-hexyle.

On mentionne que selon une variante particulière de l'un ou de l'autre des modes de réalisations particuliers de l'invention, le composé de l'invention est un composé différent des composés suivants :
- MeOOC-CHEt-CH₂-CONMe₂
- MeOOC-CH₂-CH(CH₃)-CH₂-CONMe₂
- MeOOC-CH₂-CH₂-CH₂-CONMe₂
- MeOOC-CH₂-CH₂-CONMe₂
si ces derniers ne sont pas utilisés en mélange avec d'autres composés répondant à la formule (l).

On mentionne que selon une variante encore plus particulière de l'un ou de l'autre des modes de réalisations particuliers de l'invention, le composé de l'invention est un composé nouveau de l'invention, différent des composés ou mélanges suivants, si ces derniers, individuellement, ne sont pas utilisés en mélange avec d'autres composés répondant à la formule (I) :
- MeOOC-CHEt-CH₂-CONMe₂
- MeOOC-CH₂-CH(CH₃)-CH₂-CONMe₂
- MeOOC-CH₂-CH₂-CH₂-CONMe₂
- MeOOC-CH₂-CH₂-CONMe₂
- mélange de PhOOC-CH(CH₃)-CH₂-CONEt₂ et PhOOC-CH₂-CH₂-CH₂-CONEt₂
- EtOOC-CH(CH₃)-CH₂-CONEt₂
- MeOOC-CH(CH₃)-CH₂-CONEt₂
- Me-CH(OMe)-OOC-CH(CH₃)-CH₂-CONEt₂
- Cyclohexyl-OOC-CH(CH₃)-CH₂-CONEt₂
- Ph-CH₂OOC-CH(CH₃)-CH₂-CONEt₂
- p-cresyl―OOC-CH(CH₃)-CH₂-CONEt₂
- mélange de EtOOC-CHEt-CH₂-CONEt₂, EtOOC-CH(CH₃)-CH₂-CH₂-CONEt₂ et EtOOC-CH₂-CH₂-CH₂-CH₂-CONEt₂,
- MeOOC-CH₂-CH(CH₃)-CH₂-CONH(n-butyle).

On mentionne que selon une variante encore plus particulière de l'un ou de l'autre des modes de réalisations particuliers de l'invention, le composé de l'invention est un composé nouveau de l'invention, différent des composés ou mélanges suivants, si ces derniers, individuellement, ne sont pas utilisés en mélange avec d'autres composés répondant à la formule (I) :
C₄H₉-OOC-CH₂-CH₂-CONEt₂
C₆H₁₃-OOC-(CH₂)₈-CON(C₃H₇)₂
C₈H₁₇-OOC-(CH₂)₈-CON(C₄H₉)₂
C₈H₁₇-OOC-(CH₂)₈-CON(C₈H₁₇)₂.

On mentionne qu'on peut utiliser les composés suivants en mélange avec d'autres composés répondant à la formule (I) :
- MeOOC-CHEt-CH₂-CONMe₂
- MeOOC-CH₂-CH(CH₃)-CH₂-CONMe₂
- MeOOC-CH₂-CH₂-CH₂-CONMe₂
- MeOOC-CH₂-CH₂-CONMe₂
- mélange de PhOOC-CH(CH₃)-CH₂-CONEt₂ et PhOOC-CH₂-CH₂-CH₂-CONEt₂
- EtOOC-CH(CH₃)-CH₂-CONEt₂
- MeOOC-CH(CH₃)-CH₂-CONEt₂
- Me-CH(OMe)-OOC-CH(CH₃)-CH₂-CONEt₂
- Cyclohexyl-OOC-CH(CH₃)-CH₂-CONEt₂
- Ph-CH₂OOC-CH(CH₃)-CH₂-CONEt₂
- p-cresyl―OOC-CH(CH₃)-CH₂-CONEt₂
- mélange de EtOOC-CHEt-CH₂-CONEt₂, EtOOC-CH(CH₃)-CH₂-CH₂-CONEt₂ et EtOOC-CH₂-CH₂-CH₂-CH₂-CONEt₂,
- MeOOC-CH₂-CH(CH₃)-CH₂-CONH(n-butyle).
- C₄H₉-OOC-CH₂-CH₂-CONEt₂
- C₆H₁₃-OOC-(CH₂)₈-CON(C₃H₇)₂
- C₈H₁₇OOC-(CH₂)₈-CON(C₄H₉)₂
- C₈H₁₇-OOC-(CH₂)₈-CON(C₈H₁₇)_{2.}

On mentionne que selon une variante toujours plus particulière de l'un ou de l'autre des modes de réalisations particuliers de l'invention, on n'utilise pas les composés ou mélanges suivants:
- MeOOC-CHEt-CH₂-CONMe₂
- MeOOC-CH₂-CH(CH₃)-CH₂-CONMe₂
- MeOOC-CH₂-CH₂-CH₂-CONMe₂
- MeOOC-CH₂-CH₂-CONMe₂
- mélange de PhOOC-CH(CH₃)-CH₂-CONEt₂ et PhOOC-CH₂-CH₂-CH₂-CONEt₂
- EtOOC-CH(CH₃)-CH₂-CONEt₂
- MeOOC-CH(CH₃)-CH₂-CONEt₂
- Me-CH(OMe)-OOC-CH(CH₃)-CH₂-CONEt₂
- Cyclohexyl-OOC-CH(CH₃)-CH₂-CONEt₂
- Ph-CH₂OOC-CH(CH₃)-CH₂-CONEt₂
- p-cresyl―OOC-CH(CH₃)-CH₂-CONEt₂
- mélange de EtOOC-CHEt-CH₂-CONEt₂, EtOOC-CH(CH₃)-CH₂-CH₂-CONEt₂ et EtOOC-CH₂-CH₂-CH₂-CH₂-CONEt₂,
- MeOOC-CH₂-CH(CH₃)-CH₂-CONH(n-butyle).

On mentionne que selon une variante toujours plus particulière de l'un ou de l'autre des modes de réalisations particuliers de l'invention, on n'utilise pas les composés ou mélanges suivants:
- C₄H₉-OOC-CH₂-CH₂-CONEt₂
- C₆H₁₃-OOC-(CH₂)₈-CON(C₃H₇)₂
- C₈H₁₇-OOC-(CH₂)₈-CON(C₄H₉₎₂
- C₈H₁₇-OOC-(CH₂)₈-CON(C₈H₁₇)_{2.}

Selon un mode de réalisation avantageux l'esteramide a un point de fusion inférieur à ou égal à 20°C, de préférence à 5°C, de préférence à 0°C.

### Procédé

Le composé de l'invention peut être préparé par toute méthode appropriée. On peut notamment mettre en oeuvre une étape de réaction d'un anhydride de formule (1'), avec un alcool de formule R¹-OH et/ou une amine de formule HNR²R³

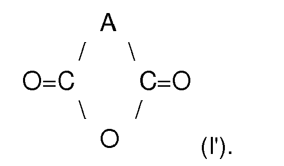

L'anhydride peut être préparé lors d'une étape a) préalable de cyclisation d'un diacide de formule HOOC-A-COOH, de préférence par réaction du diacide avec de l'anhydride acétique. On peut notamment opérer un reflux dans un excès d'anhydride acétique. Ensuite on peut opérer une condensation du produit de formule (I').

On peut notamment mettre en oeuvre l'une des séquences réactionnelles 1) ou 2) suivantes:
Séquence1):
   Etape 1b) on fait réagir l'anhydride de formule (I') avec un alcool de formule R¹-OH, de manière à obtenir un composé ester-acide de formule (I") R¹-OOC-A-COOH,
   Etape 1c) on transforme le composé de formule (I") en composé de formule (I) à l'aide d'une amine de formule HNR²R³,
Séquence 2):
   Etape 2b) on fait réagir l'anhydride de formule (I') avec une amine de formule HNR²R³ de manière à obtenir un composé amide-acide de formule (ll")

      HOOC-A-CONR²R³ (ll')

      ,
   Etape 2c) on transforme le composé de formule (ll") en composé de formule (l) à l'aide d'un alcool de formule R¹-OH.

L'étape 1b) est de préférence mise en oeuvre à l'aide d'au moins 1 équivalent molaire d'alcool, par rapport à l'anhydride. On peut mettre en oeuvre un fort excès d'alcool, par exemple de 2 à 20 équivalents, notamment de 5 à 15. On peut notamment utiliser l'alcool comme solvant de la réaction.

Selon un mode de réalisation particulier l'étape 1c) comprend les étapes suivantes (qui peuvent être simultanées ou subséquentes, de préférence subséquentes) :
1c1) on transforme le composé de formule (l") en un chlorure d'acyle de formule (l"') suivante, de préférence par réaction avec du chlorure de thionyle,

   R¹-OOC-A-COCl (l"')
1c2) on fait réagir le composé de formule (l"') avec l'amine de formule HNR³R⁴ de manière à obtenir le composé de formule (l).

L'étape 1c2) s'accompagne de formation d'acide chlorhydrique. On peut utiliser un base afin de le piéger, par exemple de la triéthanolamine ou triéthylamine (TEA). Cette étape peut être mise en oeuvre avec au moins 0,8 équivalent molaire d'amine, de préférence avec au moins un équivalent. On peut notamment mettre en oeuvre un excès de 1,05 à 1,4 équivalents molaires.

Selon un autre procédé utile pour préparer le composé de l'invention, on met en oeuvre une étape de réaction d'un diester de formule R¹OOC-A-COOR¹ avec une amine de formule HNR²R³, puis éventuellement une étape de réaction avec un alcool de formule R^{1'}-OH, où R^{1'} est un groupe choisi parmi les groupes R¹ mentionnés plus haut, mais différent du groupe R¹ du diester. Ce procédé est particulièrement intéressant et économique car les diesters sont préparés en grandes quantités et facilement disponibles. Il est ainsi possible d'optimiser les processus de production. On peut par exemple mettre en oeuvre la séquence réactionnelle 3) suivante:
Séquence 3)
   Etape 3a) on fait réagir un diester de formule R¹OOC-A-COOR¹, de préférence de formule MeOOC-A_{MG}-COOMe ou MeOOC-A_{ES}-COOMe avec une amine de formule HNR²R³ de manière à obtenir un produit comprenant un esteramide de formule R¹OOC-A-CONR²R³,
   de préférence R¹OOC-A_{MG}-CONR²R³ ou R¹OOC-A_{ES}-CONR²R³, de préférence MeOOC-A_{MG}-CONR²R³ ou MeOOC-A_{ES}-CONR²R³
   Etape 3b) éventuellement, on fait réagir avec un alcool de formule R^{1'}-OH de manière à obtenir un produit comprenant un esteramide de formule R^{1'}OOC-A-CONR²R³
   de préférence R^{1'}OOC-A_{MG}-CONR²R³ ou R^{1'}OOC-A_{ES}-CONR²R³
   où R^{1'} est un groupe choisi parmi les groupes R¹ mentionnés plus hauts, mais différent du groupe R¹ du diester.

Si le diester de départ présente le groupe R¹ du composé souhaité, alors l'étape 3b) est généralement inutile. Sinon on mettra typiquement en oeuvre cette étape. De préférence on part du diester présentant le groupe R¹ souhaité.

Lors de l'étape 3a) on met de préférence en oeuvre de 0,7 à 1,5, par exemple 0,8 à 1,2 moles, de préférence de 0,9 à 1,1 moles, de préférence environ 1 mole, d'amine par mole de diester. Il est avantageux d'opérer avec un léger excès comme un excès d'au moins 1,05 mole d'amine par mole de diester, par exemple de 1,05 à 1,1 mole d'amine par mole de diester.

L'étape 3a) peut être mise en oeuvre en solution, par exemple en solution aqueuse, ou en solution dans un solvant comme du toluène ou un alcool. On préfère opérer dans en solution non aqueuse, en évitant toute présence d'eau. On peut au cours de cette étape éliminer progressivement le méthanol formé afin de favoriser la réaction. L'élimination peut s'accompagner d'une élimination du solvant, par exemple à un azéotrope. Après séparation du méthanol le solvant éliminé peut être réintroduit dans le procédé. L'étape 3a) est de préférence mise en oeuvre en présence d'un catalyseur, en particulier d'un catalyseur de type basique. On peut par exemple utiliser des méthylates comme MeONa, des carbonates comme K₂CO₃, Na₂CO₃, des titanates.

L'étape 3b) est une étape de trans-esterification. Elle peut notamment être catalysée par des acides ou des bases, par exemple par K₂CO₃, ou Na₂CO₃

On note que dans tous les procédés et séquences mentionnés ci-dessus, on peut mettre en oeuvre des étapes optionnelles intermédiaires de séparation et/ou de purification, afin d'éliminer des sous-produits non visés. Les sous-produits peuvent être éventuellement utilisés pour fabriquer d'autres produits, ou peuvent être transformés afin d'être re-introduits dans le procédé.

La réaction peut être suivie d'étapes de filtration et/ou de purification par exemple par distillation.

Les diacides, le cas échéant sous forme de mélanges, peuvent notamment être obtenus à partir d'un mélange de composés dinitriles, le cas échéant sous forme de mélanges. Les dinitriles peuvent notamment être des dinitriles produits et récupérés dans le procédé de fabrication de l'adiponitrile par double hydrocyanation du butadiène. Dans ce cas il peut s'agir de mélanges de dinitriles. Ce procédé utilisé à grande échelle dans l'industrie pour produire la grande majorité de l'adiponitrile consommé dans le monde est décrit dans de nombreux brevets et ouvrages.

La réaction d'hydrocyanation du butadiène conduit majoritairement à la formation de dinitriles linéaires mais également à une formation de dinitriles ramifiés dont les deux principaux sont le méthylglutaronitrile et l'éthylsuccinonitrile.

Dans les étapes de séparation et de purification de l'adiponitrile, les composés dinitriles ramifiés sont séparés par distillation et récupérés, par exemple, comme fraction de tête dans une colonne de distillation.

Des diacides utiles peuvent être obtenus par réaction entre les composés dinitriles et une base minérale, pour obtenir des sels d'acide, puis neutralisation de ces sels par un acide. Des diacides utiles peuvent également être obtenus par hydrolyse acide des composés dinitriles.

Des diesters de formule R¹OOC-A-COOR¹ utiles pour la mise en oeuvre de la séquence 3 sont disponible dans le commerce, notamment auprès de Invista sous les références DBE, ou auprès de Rhodia sous le nom Rhodiasolv® RPDE.

Des procédés de préparation de diacides et/ou de diesters sont notamment décrits dans les documents W02007/101929, FR 2902095, WO 2008/009792, WO 2008/062058.

### Utilisations - Formulations

Le composé de l'invention et/ou une composition de matière le comprenant décrite ci-dessus, peut notamment être utilisé comme solvant, co-solvant et/ou inhibiteur de cristallisation ou comme agent de coalescence.

Par co-solvant, on entend que d'autres solvants peuvent lui être associés. L'utilisation à titre de solvant ou de co-solvant comprend notamment l'utilisation pour dissoudre un composé dans une formulation, dans un milieu réactionnel, l'utilisation pour solubiliser totalement ou partiellement un produit à éliminer (dégraissage, décapage), et/ou pour faciliter de décollage de films de matières.

Le composé de l'invention et/ou une composition de matière le comprenant décrite ci-dessus, peut notamment être utilisé, pour les fonctions indiquées ci-dessus ou pour d'autres, dans une formulation phytosanitaire, dans une formulation de nettoyage, dans une formulation de décapage, dans une formulation de dégraissage, dans une formulation de lubrifiants ou textiles, dans une formulation de revêtement, par exemple dans une formulation de peinture, dans une formulation de pigments ou encre.

Le composé peut par exemple être utilisé à titre d'agent de coalescence dans une formulation de peinture aqueuse.

Le composé peut notamment être utilisé comme agent de dégraissage sur des surfaces métalliques, par exemple des surfaces d'outils, d'objets manufacturés, de tôles, de moules, notamment en acier ou en aluminium ou en alliages de ces métaux.

Le composé peut notamment être utilisé comme solvant de nettoyage sur des surfaces dures ou des surfaces textiles.

Le composé peut notamment être utilisé comme solvant de décapage de peinture ou de résines, sur des surfaces d'outils, par exemple des moules de fonderie, sur des surfaces des sites industriels (sols, cloisons etc...).

Les formulations de nettoyage et/ou de dégraissage peuvent notamment être des formulations pour les soins ménagers, opérés dans les foyers ou dans les domaines publiques (hôtels, bureaux, usines....). Il peut s'agir de formulations pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyés.

Dans le cadre d'utilisations dans des milieux réactionnels, on cite notamment les utilisation dans le cadre de polymérisations en solution, notamment pour la préparation en solution de polycondensats, notamment de polymides ou de polyesters ou de polyamides ou de polyamide-imides, notamment de polycondensats partiellement ou totalement aromatiques comme des polyamides aromatiques (aramides).

Le composé de l'invention et/ou une composition de matière le comprenant décrite ci-dessus, peut notamment être utilisé dans des formulations phytosanitaires comprenant un produit actif solide. Plus de détails sont donnés ci-dessous, où le mot "solvant" peut désigner le composé de l'invention ou une composition de matière le comprenant, décrite ci-dessus.

### Utilisation détaillée dans le cadre de formulations phytosanitaires

La formulation phytosanitaire est généralement une formulation phytosanitaire concentrée comprenant un composé actif.

L'agriculture utilise de nombreuses matières actives telles que des fertilisants ou des pesticides, par exemple des insecticides, herbicides ou fongicides. On parle de produits phytosanitaires actifs (ou de matière active). Les produits phytosanitaires actifs sont généralement produits sous forme pure ou très concentrée. Ils doivent être utilisés sur les exploitations agricoles à de faibles concentrations. A cette fin, ils sont généralement formulés avec d'autres ingrédients afin de permettre une dilution en poids aisée par l'exploitant agricole. On parle de formulations phytosanitaires. La dilution opérée par l'exploitant agricole est généralement réalisée par mélange de la formulation phytosanitaire avec de l'eau.

Ainsi les formulations phytosanitaires doivent permettre une dilution en poids aisée par l'exploitant agricole, afin d'obtenir un produit dans lequel le produit phytosanitaire est correctement dispersé, par exemple sous forme de solution, d'émulsion, de suspension, ou de suspo-émulsion. Les formulations phytosanitaires permettent ainsi le transport d'un produit phytosanitaire sous forme relativement concentrée, un conditionnement aisé et/ou une manipulation aisée pour l'utilisateur final. Différents types de formulations phytosanitaires peuvent être utilisés selon les différents produits phytosanitaires. On cite par exemple les concentrés émulsionnables (Emulsifiable Concentrates «EC»), les émulsions concentrées (Emulsion in water "EW"), les microémulsions ("ME"), les poudres mouillables (Wettable Powders «WP»), les granulés dispersables dans l'eau (Water Dispersible Granules, «WDG»). Les formulations qu'il est possible d'utiliser dépendent de la forme physique du produit phytosanitaire (par exemple solide ou liquide), et de ses propriétés physico-chimiques en présence d'autres composés comme l'eau ou les solvants.

Après dilution en poids par l'exploitant agricole, par exemple par mélange avec de l'eau, le produit phytosanitaire peut se trouver sous différentes formes physiques: solution, dispersion de particules solides, dispersion de gouttelettes du produit, gouttelettes de solvant dans lequel le produit est dissous... Les formulations phytosanitaires comprennent généralement des composés permettant d'obtenir ces formes physiques. Il peut par exemple s'agir de tensioactifs, de solvants, de supports minéraux, et/ou de dispersants. Bien souvent ces composés n'ont pas un caractère actif, mais un caractère d'intermédiaire d'aide à la formulation. Les formulations phytosanitaires peuvent notamment être sous forme liquide, ou sous forme solide.

Afin de préparer des formulations phytosanitaires de produits phytosanitaires actifs solides, il est connu de solubiliser le produit dans un solvant. La formulation phytosanitaire comprend ainsi une solution du produit dans le solvant. La formulation peut être sous forme solide, par exemple sous forme de poudre mouillable (WP) où la solution imbibe un support inorganique, par exemple du kaolin et/ou de la silice. La formulation peut alternativement être sous forme liquide, par exemple sous forme de concentré émulsionnable (EC) présentant une seule phase liquide limpide comprenant le solvant et le produit en solution, pouvant former une émulsion par ajout d'eau, sans agitation ou avec une faible agitation. Elle peut aussi être ou sous forme d'une émulsion concentrée (EW), trouble, dont la phase dispersée dans l'eau comprend le solvant et le produit en solution dans le solvant. Elle peut aussi être forme d'une microémulsion (ME), limpide, dont la phase dispersée dans l'eau comprend le solvant et le produit en solution dans le solvant.

Certains actifs phytosanitaires solides sont souvent difficiles à formuler. Par exemple le tebuconazole est un fongicide particulièrement efficace, et d'utilisation répandue, pour la culture du soja notamment. Pour certains actifs phytosanitaires, il est difficile de réaliser des formulations concentrées, faciles à diluer pour l'exploitant agricole, stables, et sans inconvénients (avérés ou perçus) substantiels en matière de sécurité, de toxicité et/ou d'eco-toxicité. Pour certains actifs, il est difficile de formuler à des concentrations relativement élevées, avec une stabilité suffisante. En particulier il est nécessaire d'éviter l'apparition de cristaux en particulier à basse température et/ou lors de la dilution et/ou lors du stockage à température élevée de la composition diluée. Les cristaux peuvent avoir des effets négatifs, notamment boucher les filtres des dispositifs utiliser pour répandre la composition diluée, boucher les dispositifs de pulvérisation, diminuer l'activité globale de la formulation, créer des problèmes inutiles de filières de déchets pour éliminer les cristaux, et/ou provoquer une mauvaise répartition du produit actif sur le champ agricole.

Les formulations comprenant le solvant présentent notamment:
- une solubilisation de quantités importantes d'actifs,
- une absence de cristallisation, même des conditions exigeantes,
- une bonne activité biologique pouvant être due à une bonne solvatation, et/ou
- un profile de sécurité, toxicologie et/ou eco-toxicologie perçu comme favorable.

La formulation phytosanitaire peut en outre être une formulation phytosanitaire concentrée comprenant:
a) un produit phytosanitaire actif,
b) le solvant (composé esteramide)
c) éventuellement au moins agent émulsifiant, de préférence un tensioactif,
   et
d) éventuellement de l'eau.

### Produit phytosanitaire actif a)

Des produits phytosanitaires actifs, notamment des produits non solubles dans l'eau et solides sont connus de l'homme du métier. Le produit phytosanitaire actif peut notamment être un herbicide, un insecticide, un acaricide, un fongicide, ou un agent d'élimination des rongeurs ("rodenticide" en anglais) par exemple un raticide.

A titre d'exemples non limitatifs de matières actives convenables, on peut citer entre autres l'Amétryne, le Diuron, le Linuron, le Chlortoluron, l'Isoproturon, le Nicosulfuron, le Metamitron, le Diazinon, l'Aclonifen, l'Atrazine, le Chlorothalonil, le Bromoxynil, le Bromoxynil heptanoate, le Bromoxynil octanoate, le Mancozeb, la Manèbe, le Zineb, la Phenmédipham, le Propanyl, la série des phénoxyphénoxy, la série des hétéroaryloxyphénoxy, le CMPP, le MCPA, le 2,4-D, la Simazine, les produits actifs de la série des imidazolinones, la famille des organophosphorés, avec notamment l'Azinphos-éthyl, l'Azinphos-méthyl, l'Alachlore, le Chlorpyriphos, le Diclofop-méthyl, le Fénoxaprop-p-éthyl, le Méthoxychlore, la Cyperméthrine, le Fenoxycarbe, le cymoxanil, le chlorothalonyl, Ikes insecticides neonicotinoides, la famille des fongicide triazoles tels que l'azaconazole, bromuconazole, cyproconazole, difenoconazole, diniconazole, epoxyconazole, fenbuconazole, flusilazole, myclobutanyl, tebuconazole, triadimefon, triadimenol, des strobilurines telles que la pyraclostrobine, la picoxystrobine, l'azoxystrobine, la famoxadone, le kresoxym-methyl et la trifloxystrobine, les solfonylurées telles que le bensulfuron-methyl, le chlorimuron-ethyl, le chlorsulfuron, le metsulfuron-methyl, le nicosulfuron, le sulfomethuron-methyl, le triasulfuron, le tribenuron-methyl.

On choisit parmi cette liste les produits non-hydrosolubles.

On peut notamment mettre en oeuvre les produits phytosanitaires actifs suivants :

| | |
|---|---|
| Alachlor | 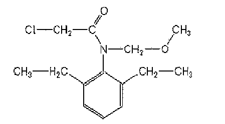 |
| Chlorpyrifos | 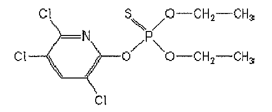 |
| alphacyperméthrine | 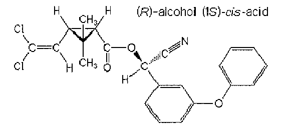 |
| | 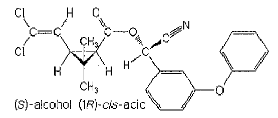 |
| | En mélange racémique et/ou en stéréoisomères isolés. |
| Phenmedipham | 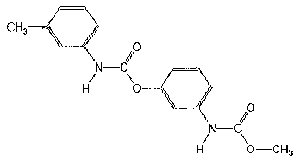 |
| Propanil | 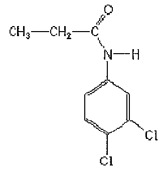 |
| Pendimethalin | 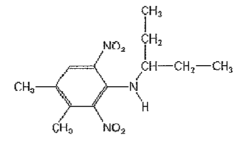 |
| triadimenol | 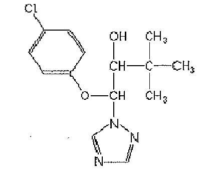 |
| Trifluralin | 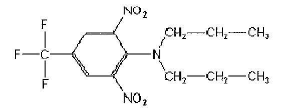 |
| Oxyfluorfen | 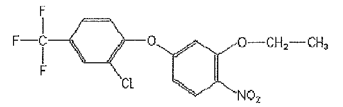 |
| Dimethoate | 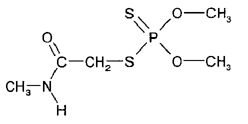 |
| Imidacloprid | 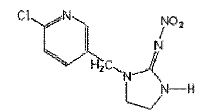 |
| Proxopur | 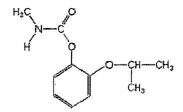 |
| Benomyl | 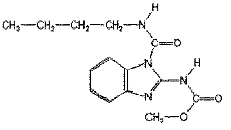 |
| Deltamethrine | 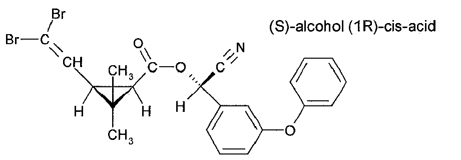 |
| Fenvalerate | 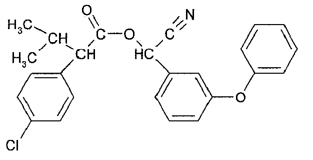 |
| Abamectin | 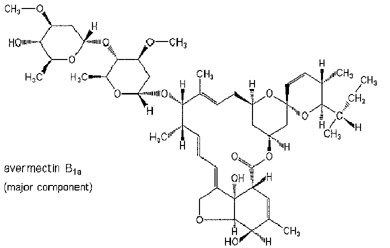 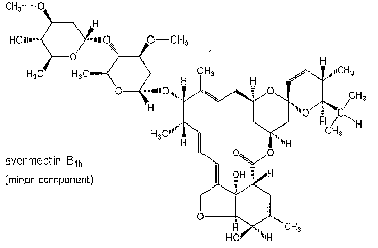 |
| Amicarbazone | 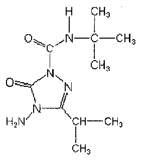 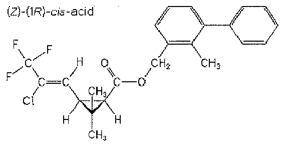 |
| Bifenthrin | 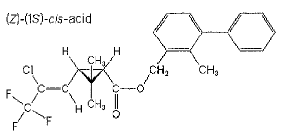 |
| Carbosulfan | 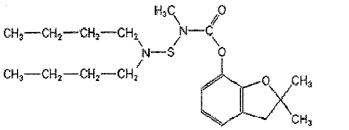 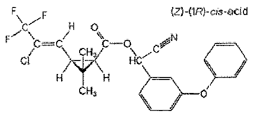 |
| Cyfluthrin | 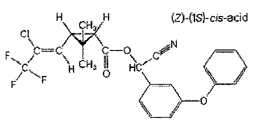 |
| Difenconazole | 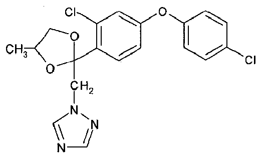 |
| Ethofenprox | 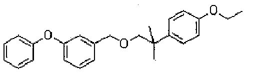 |
| Fenoxapropethyl | 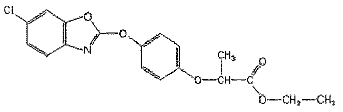 |
| Fipronil | 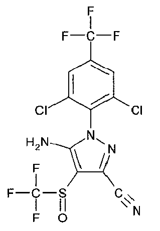 |
| Fenvalerate | 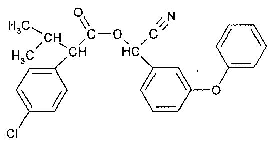 |
| Fluazifop-p-butyl | 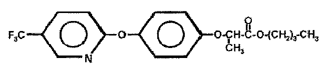 |
| Flufenouron | 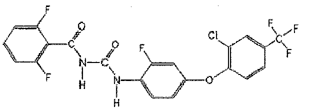 |
| Hexazinone | 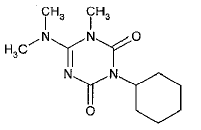 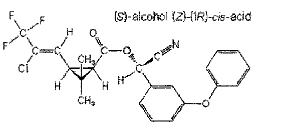 |
| Lambdacyalothrin | 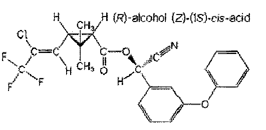 |
| Methomyl | 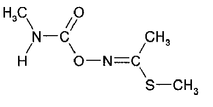 |
| Permethrin | 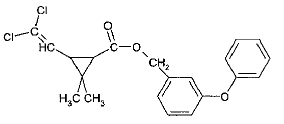 |
| Prochloraz | 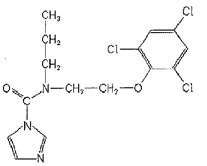 |
| Propiconazole | 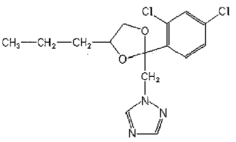 |
| Tebuconazole | 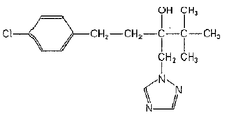 |

Ces produits et dénominations sont connus de l'homme du métier. On peut associer plusieurs produits phytosanitaires actifs.

### Agent émulsifiant c)

La formulation phytosanitaire peut comprendre un agent émulsifiant, typiquement et de préférence un tensioactif. Les agents émulsifiants sont des agents destinés à faciliter la mise en émulsion ou la dispersion après mise en présence de la formulation avec de l'eau, et/ou à stabiliser (dans le temps et/ou en température) l'émulsion ou la dispersion, par exemple en évitant une sédimentation.

Les tensioactifs sont des composés connus, qui présentent une masse molaire généralement relativement faible, par exemple inférieure à 1000 g/mol. Le tensioactif peut être un tensioactif anionique sous forme salifiée ou acide, non ionique de préférence polyalcoxylé, cationique, amphotère (terme incluant aussi les tensioactifs zwitterioniques). Il peut s'agir d'un mélange ou d'une association de ces tensioactifs.

A titre d'exemples de tensioactifs anioniques, on peut citer, sans intention de s'y limiter :
- les acides alkylsulfoniques, les acides arylsulfoniques, éventuellement substitués par un ou plusieurs groupements hydrocarbonés, et dont la fonction acide est partiellement ou totalement salifiée, comme les acides alkylsulfoniques en C₈-C₅₀, plus particulièrement en C₈-C₃₀, de préférence en C₁₀-C₂₂, les acides benzènesulfoniques, les acides naphtalènesulfoniques, substitués par un à trois groupements alkyles en C₁-C₃₀, de préférence en C₄-C₁₆, et/ou alcényles en C₂-C₃₀, de préférence en C₄-C₁₆.
- les mono- ou diesters d'acides alkylsulfosucciniques, dont la partie alkyle, linéaire ou ramifiée, éventuellement substituée par un ou plusieurs groupements hydroxylés et/ou alcoxylés, linéaires ou ramifiés en C₂-C₄ (de préférence éthoxylés, propoxylés, éthopropoxylés).
- les esters phosphates choisis plus particulièrement parmi ceux comprenant au moins un groupement hydrocarboné saturé, insaturé ou aromatique, linéaire ou ramifié, comprenant 8 à 40 atomes de carbone, de préférence 10 à 30, éventuellement substitués par au moins un groupement alcoxylé (éthoxylé, propoxylé, éthopropoxylé). En outre, ils comprennent au moins un groupe ester phosphate, mono- ou diestérifié de telle sorte que l'on puisse avoir un ou deux groupes acides libres ou partiellement ou totalement salifiés. Les esters phosphates préférés sont du type des mono- et diesters de l'acide phosphorique et de mono-, di- ou tristyrylphénol alcoxylé (éthoxylé et/ou propoxylé), ou de mono-, di- ou trialkylphénol alcoxylé (éthoxylé et/ou propoxylé), éventuellement substitué par un à quatre groupements alkyles ; de l'acide phosphorique et d'un alcool en C₈-C₃₀, de préférence en C₁₀-C₂₂ alcoxylé (éthoxylé ou éthopropoxylé); de l'acide phosphorique et d'un alcool en C₈-C₂₂, de préférence en C₁₀-C₂₂, non alcoxylé.
- les esters sulfates obtenus à partir d'alcools saturés, ou aromatiques, éventuellement substitués par un ou plusieurs groupements alcoxylés (éthoxylés, propoxylés, éthopropoxylés), et pour lesquels les fonctions sulfates se présentent sous la forme acide libre, ou partiellement ou totalement neutralisées. A titre d'exemple, on peut citer les esters sulfates obtenus plus particulièrement à partir d'alcools en C₈-C₂₀, saturés ou insaturés, pouvant comprendre 1 à 8 motifs alcoxylés (éthoxylés, propoxylés, éthopropoxylés) ; les esters sulfates obtenus à partir de phénol polyalcoxylé, substitués par 1 à 3 groupements hydroxycarbonés en C₂-C₃₀, saturés ou insaturés, et dans lesquels le nombre de motifs alcoxylés est compris entre 2 et 40 ; les esters sulfates obtenus à partir de mono-, di- ou tristyrylphénol polyalcoxylés dans lesquels le nombre de motifs alcoxylés varie de 2 à 40.

Les tensioactifs anioniques peuvent être sous forme acide (il sont potentiellement anioniques), ou sous une forme partiellement ou totalement salifiée, avec un contre-ion. Le contre-ion peut être un métal alcalin, tel que le sodium ou le potassium, un alcalino-terreux, tel que le calcium, ou encore un ion ammonium de formule N(R)₄⁺ dans laquelle R, identiques ou différents, représentent un atome d'hydrogène ou un radical alkyle en C₁-C₄ éventuellement substitué par un atome d'oxygène.

A titres d'exemples de tensioactifs non ioniques, on peut citer, sans intention de s'y limiter:
- les phénols polyalcoxylés (éthoxylés, propoxylés, éthopropoxylés) substitués par au moins un radical alkyle en C₄-C₂₀, de préférence en C₄-C₁₂, ou substitués par au moins un radical alkylaryle dont la partie alkyle est en C₁-C₆. Plus particulièrement, le nombre total de motifs alcoxylés est compris entre 2 et 100. A titre d'exemple, on peut citer les mono-, di- ou tri (phényléthyl) phénols polyalcoxylés, ou les nonylphénols polyalcoxylés. Parmi les di- ou tristyrylphenols éthoxylés et/ou propoxylés, sulfatés et/ou phosphatés, on peut citer, le di-(phényl-1 éthyl)phénol éthoxylé, contenant 10 motifs oxyéthylénés, le di-(phényl-1 éthyl)phénol éthoxylé, contenant 7 motifs oxyéthylénés, le di-(phényl-1 éthyl)phénol éthoxylé sulfaté, contenant 7 motifs oxyéthylénés, le tri-(phényl-1 éthyl)phénol éthoxylé, contenant 8 motifs oxyéthylénés, le tri-(phényl-1 éthyl)phénol éthoxylé, contenant 16 motifs oxyéthylénés, le tri-(phényl-1 éthyl)phénol éthoxylé sulfaté, contenant 16 motifs oxyéthylénés, le tri-(phényl-1 éthyl)phénol éthoxylé, contenant 20 motifs oxyéthylénés, le tri-(phényl-1 éthyl)phénol éthoxylé phosphaté, contenant 16 motifs oxyéthylénés.
- les alcools ou les acides gras en C₆-C₂₂, polyalcoxylés (éthoxylés, propoxylés, éthopropoxylés). Le nombre des motifs alcoxylés est compris entre 1 et 60. Le terme acide gras éthoxylé inclut aussi bien les produits obtenus par éthoxylation d'un acide gras par l'oxyde d'éthylène que ceux obtenus par estérification d'un acide gras par un polyéthylèneglycol.
- les triglycérides polyalcoxylés (éthoxylés, propoxylés, éthopropoxylés) d'origine végétale ou animale. Ainsi conviennent les triglycérides issus du saindoux, du suif, de l'huile d'arachide, de l'huile de beurre, de l'huile de graine de coton, de l'huile de lin, de l'huile d'olive, de l'huile de palme, de l'huile de pépins de raisin, de l'huile de poisson, de l'huile de soja, de l'huile de ricin, de l'huile de colza, de l'huile de coprah, de l'huile de noix de coco, et comprenant un nombre total de motifs alcoxylés compris entre 1 et 60. Le terme triglycéride éthoxylé vise aussi bien les produits obtenus par éthoxylation d'un triglycéride par l'oxyde d'éthylène que ceux obtenus par trans-estérification d'un triglycéride par un polyéthylèneglycol.
- les esters de sorbitan éventuellement polyalcoxylés (éthoxylés, propoxylés, éthopropoxylés), plus particulièrement les esters de sorbitol cyclisé d'acides gras de C₁₀ à C₂₀ comme l'acide laurique, l'acide stéarique ou l'acide oléique, et comprenant un nombre total de motifs alcoxylés compris entre 2 et 50.

Des émulsifiants utiles sont notamment les produits suivants, tous commercialisés par Rhodia :
- Soprophor® TSP/724: tensioactif à base de tristyrylphonol éthopropoxylé
- Soprophor® 796/O: tensioactif à base de tristyrylphonol éthopropoxylé
- Soprophor® CY 8: tensioactif à base de tristyrylphonol éthoxylé
- Soprophor® BSU: tensioactif à base de tristyrylphonol éthoxylé
- Alkamuls® RC: tensioactif à base d'huile de ricin éthoxylée
- Alkamuls® OR/36: tensioactif à base d'huile de ricin éthoxylée
- Alkamuls® T/20: tensioactif à base d'un ester de sorbitan

La formulation comprend avantageusement au moins 4%, de préférence au moins 5%, de préférence au moins 8%, en poids de matière sèche, d'au moins un tensioactif c).

On mentionne que le solvant peut être associé à un tensioactif aromatique et/ou non aromatique.

### Autres détails quant à la formulation phytosanitaire

La formulation phytosanitaire, concentrée, ne comprend de préférence pas des quantités importantes d'eau. Typiquement la teneur en eau est inférieure à 50% en poids, avantageusement inférieure à 25% en poids. Elle sera généralement inférieure à 10% en poids.

La formulation est de préférence un formulation liquide, par exemple sous forme d'un concentré emulsifiable (EC), d'une émulsion concentrée (EW) ou d'une micorémulsion (ME). Dans ce cas elle comprend de préférence moins de 500 g/L d'eau, plus préférablement moins de 250 g/L. Elle sera généralement inférieure à 100 g/L.

Les formulations peuvent avantageusement comprendre:
a) de 4 à 60%, de préférence de 10 à 50%, du produit phytosanitaire, en poids de matière active,
b) de 10 à 92%, de préférence de 20 à 80%, du solvant, en poids,
c) de 4 à 60%, de préférence de 5 à 50%, de préférence de 8 à 25%, en poids de matière sèche, d'un émulsifiant, de préférence d'un tensioactif,
d) de 0 à 10% en poids d'eau.

Il n'est pas exclu de réaliser des formulations solides, par exemple des formulations dans lesquelles un liquide comprenant le produit phytosanitaire solubilisé dans le solvant, est supporté par un minéral et/ou dispersé dans une matrice solide.

La formulation peut bien entendu comprendre d'autres ingrédients (ou "autres additifs") que le produit phytosanitaire actif, le(s) solvant(s), le(s) agent(s) émulsifiant(s) optionnel(s) et l'eau optionnelle. Elle peut notamment comprendre des agents de modification de la viscosité, des agents antimousse, notamment des antimousse siliconnés, des agents anti-rebond, des agents anti-lessivage, des charges inertes, notamment des charges minérales, des agents anti-gel...

Notamment les formulations peuvent comprendre des additifs, dits autres additifs, ne rentrant pas dans la définition des produits a), b), ou c), comme:
- d'autres solvants, généralement en faible quantité, par exemple en quantité inférieure à la quantité de composé de formule (I). A titre d'autres solvants on cite notamment les solvants de la famille des phosphates, phosphonates ou des oxydes de phosphines comme le TEBP, le TBP, le TEPO, le DBBP. On cite également les alkyldiméthyleamides où l'alkyl est en C₆-C₁₈, notamment ceux commercialisés sous la marque Genagen. On cite également les lactates d'esters, notamment ceux commercilisés sous la marque Purasolv. On cite également les esters méthyliques d'acides gras, notamment ceux commercialisés sous la marque Phytorobe. On cite également les diesters de diacides ("DiBasic Esters" en anglais), notamment ceux commercialisé par Rhodia sous les marques Rhodiasolv RPDE, et Rhodiasolv DIB. On cite également les coupes d'hydrocarbures, les amides cycliques comme la NMP, les lactones. On cite également les bis(dialkylamides) décrites dans le document WO 2008//074837.
- des inhibiteurs de cristallisation. Il peut s'agir des solvants mentionnés ci-dessus. Il peut également s'agir d'acides gras ou d'alcools gras non polyalkoxylés. On cite par exemple le produit Alkamuls® OL700 commercialisé par Rhodia.

Des procédés classiques de préparation de formulations phytosanitaires ou de mélanges de solvants peuvent être mis en oeuvre. On peut opérer par simple mélange des constituants.

La formulation phytosanitaire concentrée est destinée à être répandue sur un champ cultivé ou à cultiver, par exemple un champ de soja, le plus souvent après dilution dans de l'eau, pour obtenir une composition diluée. La dilution est généralement opérée par l'exploitant agricole, directement dans un réservoir ("tank-mix"), par exemple dans le réservoir d'un dispositif destiné à répandre la composition. Il n'est pas exclu que l'exploitant ajoute d'autres produits phytosanitaires, par exemple des fongicides, herbicides, pesticides, insecticides, des fertilisants. Ainsi, la formulation peut être utilisée pour préparer une composition diluée dans l'eau du produit phytosanitaire actif, par mélange d'au moins une part en poids de formulation concentrée avec au moins 10 parts d'eau, de préférence moins de 1000 parts. Les taux de dilution et les quantités à appliquer sur le champ dépendent généralement du produit phytosanitaire et de la dose souhaitable pour traiter le champ; cela peut être déterminé par l'exploitant agricole.

D'autres détails ou avantages pourront apparaître au vu des exemples qui suivent, sans caractère limitatif.

### EXEMPLES

### Procédures générales mise en oeuvre pour la synthèse d'esteramides

### Procédure A: Formation de l'ester acide

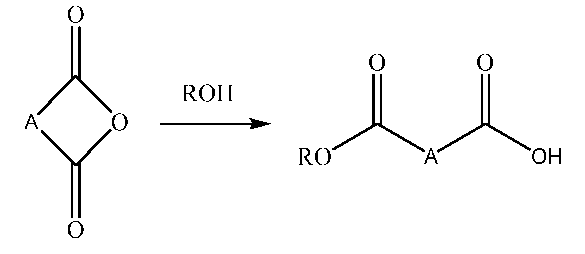

L'anhydride cyclique est mélangé avec l'alcool et chauffé a 60°C pendant 3h. Les volatiles sont retirés par distillation sous pression réduite si nécessaire. Le produit final peut être purifié par distillation sous pression réduite.

### Procédure B : Formation de l'ester / chlorure d'acide

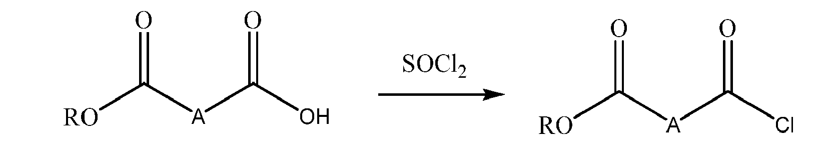

L'ester / acide et le chlorure de thionyle sont mélangés a température ambiante. Le mélange réactionnel peut être chauffé au reflux pour compléter la réaction. Les espèces volatiles sont retirées par distillation sous pression réduite pour obtenir le produit brut qui est typiquement utilise tel sans autre forme de purification.

### Procédure C : Formation de l'ester / amide

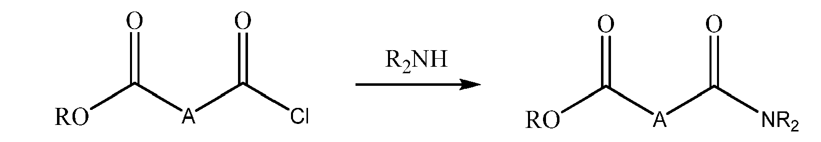

Le toluène et la triméthylamine (TEA) sont mélangés sous atmosphère inerte et refroidis à -20°C. La dimethylamine (DMA) est alors ajoutée. L'ester / chlorure d'acide est ajouté lentement de manière à maintenir la température en dessous de 0°C. Le mélange est alors agité à température ambiante pendant la nuit puis filtré pour retirer le précipité. Le filtrat est évaporé sous vide pour obtenir le produit brut. Le produit final est obtenu par distillation sous pression réduite du brut de réaction.

### Procédure D : Formation de l'amide acide

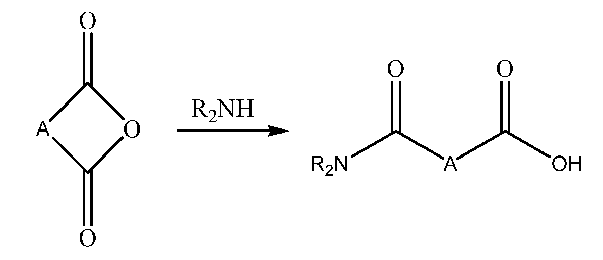

L'anhydride cyclique est ajouté à l'amine primaire en maintenant la température en dessous de 40°C. Le mélange est alors maintenu à température ambiante pendant 10-24h. Les espèces volatiles sont évaporées sous vide. Le produit peut être purifié par distillation sous pression réduite.

### Procédure E : Formation de l'esteramide

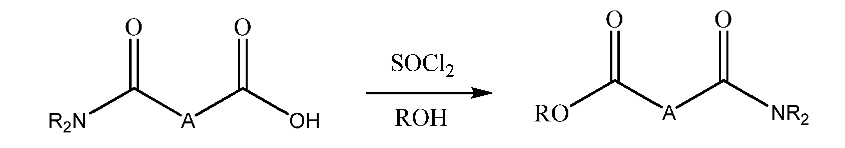

L'amide acide et l'alcool sont mélangés à température ambiante puis le chlorure de thionyle est ajouté lentement de façon à maintenir la température en dessous de 30°C. L'acide chlorhydrique formé durant la réaction peut être piégé par une solution concentrée de soude. Le mélange réactionnel est agité à température ambiante jusqu'à ce que les produits de départs aient été consommés. La réaction peut être suivie par CG. Les espèces volatiles sont évaporées sous vide pour obtenir le produit brut. Dans certains cas le brut est dissous dans le méthanol et le pH ajusté aux alentours de 6-7 avant évaporation du solvant. Le produit final est alors obtenu après distillation sous pression réduite.

### Procédure F: Préparation de l'anhydride cyclique de 2-MGA

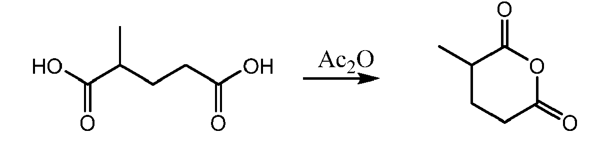

### Matières première

| Acide 2-Méthylglutarique (2-MGA) | Anhydride acétique |
|---|---|
| 327g; 2,23mol; Mw: 146,14 | 500ml; 4,9mol; 2,2eq. Mw: 102,09 |

Le 2-MGA pur et l'anhydride acétique sont mélangés et chauffés au reflux (140°C) pendant 7h. L'excès d'anhydride acétique et l'acide acétique formé sont évaporés sous vide pour obtenir le brut de réaction (320g). L'huile ainsi obtenue est distillée sous pression réduite (120°C / 310 Pa) pour obtenir un solide blanc (263g).
Rendement = 92.2%

### Exemple 1.1 - Préparation de MeOOC-CH₂-CH₂-CONMe₂

La voie de synthèse est la suivante:

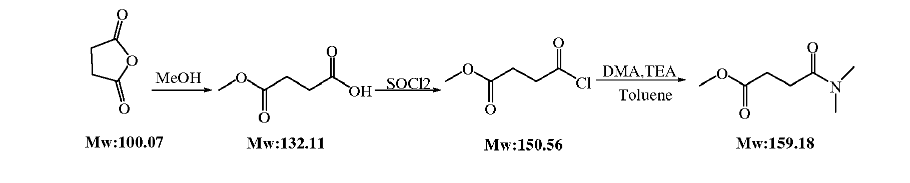

### Etape 1 :

### Matières premières

| Anhydride cyclique | Méthanol (anhydre) |
|---|---|
| 250g; 2,473mol; Mw: 100.07; 99% | 1000ml; 24,73mol, Mw: 32,04; 10eq |

L'ester acide est obtenu par la Procédure A.
Produit final = 311 g, Rendement = 94%
Analyse par CG (chromatographie gazeuse): surface > 99%

### Etape 2

### Matières premières

| Ester / acide | Chlorure de thionyle |
|---|---|
| 311 g; 2,331 mol; Mw: 132,11; | 340ml; 4,662mol; Mw: 118,97, 2eq |
| GC: (surface) = 99.03% | |

L'ester / chlorure d'acide est obtenu par la Procédure B.
Produit brut = 348g

### Etape 3

### Matières premières

| Ester / chlorure d'acide | DMA | TEA | Toluène |
|---|---|---|---|
| 348g; 2,311 mol; | 280ml; 4,22mol, | 390ml; 2,80mol, | 1750+500ml |
| 1 eq. Mw: 150,56 | 1,83eq, Mw: 45,08 | 1,2eq; Mw:101.2 | |

L'ester / amide ester obtenu par la Procédure C.
Produit brut = 402g
Produit final = 218g
Analyse CG (surface) > 98%

### Exemple 1.2 - Préparation de MeOOC-CH₂-CH₂-CH₂-CONMe₂

La voie de synthèse est la suivante:

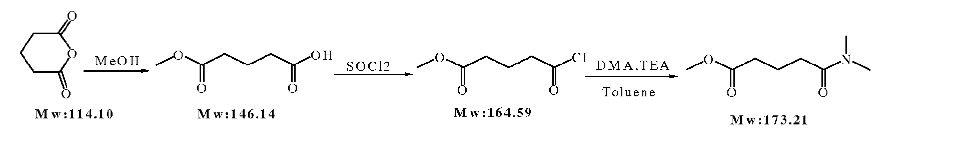

### Etape 1

### Matières premières

| Anhydride cyclique | Méthanol |
|---|---|
| 257g; 2,2mol; | 4350ml; 107,4mol; 48,8eq. Mw: 32,04 |
| 1eq. Mw: 114,10; (GC: 97.89%) | |

L'ester acide est obtenu par la Procédure A.
Produit brut = 333g (liquide jaune)
Produit final = 274g
Analyse par CG (chromatographie gazeuse) : surface > 99%

### Etape 2

### Matières premières

| Ester acide | Chlorure de thionyle |
|---|---|
| 274g; 1,869mol; 1 eq. Mw: 114,10; (GC: 99.68%) | 273ml; 3,738mol; 2eq. Mw: 118,97 |

L'ester / chlorure d'acide est obtenu par la Procédure B. Produit brut = 314g (liquide rouge), Rendement 99%

### Etape 3

### Matières premières

| Ester / chlorure d'acide | DMA | TEA | Toluène |
|---|---|---|---|
| 314g; 1,869mol; 1eq. | 250ml; 3,738mol, | 227ml; 2,243mol, | 1250+500ml |
| Mw:164,59 (si 100%) | 2eq, Mw: 45,08; | 1,2eq, Mw: 101,19; | |
| | d=0,68 | d=0,726 | |

L'ester / amide ester obtenu par la Procédure C.
Produit brut = 339g
Produit final = 237g, Rendement = 89.6%
Analyse CG (surface) > 99%

### Exemple 1.3 - Préparation de MeOOC-A_{MG}-CONMe₂ par une première voie La voie de synthèse est la suivante:

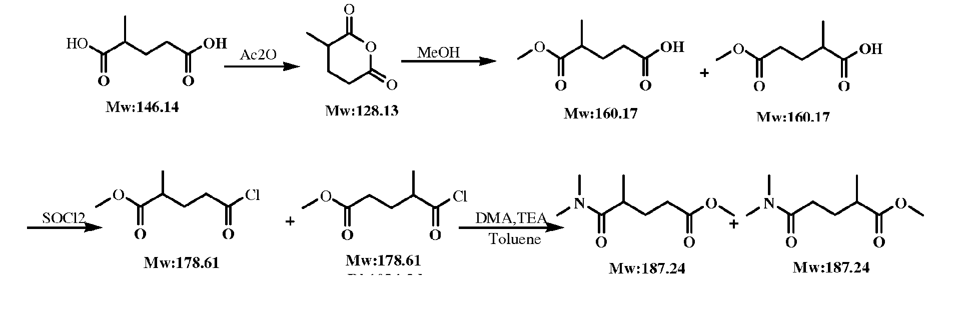

### Etape 1

### Matières premières

| Anhydride cyclique | méthanol |
|---|---|
| 245g;1,91mol; Mw: 128,13; GC > 99% | 1500ml |

L'anhydride est obtenu par la Procédure F.
L'ester acide est obtenu par la Procédure A.
Produit brut = 302g
Produit final = 261 g, Rendement = 85.6%
Analyse par CG (chromatographie gazeuse) : surface > 99% (isomères 58/42)

### Etape 2

### Matières premières

| Ester / acide | Chlorure de thionyle |
|---|---|
| 261g; 1,432mol, Mw: 160,17, | 240ml; 2,86mol; 2eq. Mw: 118,97 |

L'ester / chlorure d'acide est obtenu par la Procédure B.
Produit brut = 290g (liquide jaune)

### Etape 3

### Matières premières

| Ester / chlorure d'acide | DMA | TEA | Toluène |
|---|---|---|---|
| 290g; 1,62mol; | 218ml; 3,24mol, | 284ml; 2,02mol; 1,25eq; | 2000ml |
| Mw: 178,61 | 2eq; Mw: 45,08 | Mw: 101,19 | |

Le méthyle ester diméthyle amide est obtenu par la Procédure C.
Produit brut = 303g (liquide rouge)
Produit final, analyse CG (surface) > 99% (isomères 63/37)

### Exemple 1.4 - Préparation de MeOOC-A_{MG}-CONMe₂ par une deuxième voie La voie de synthèse est la suivante:

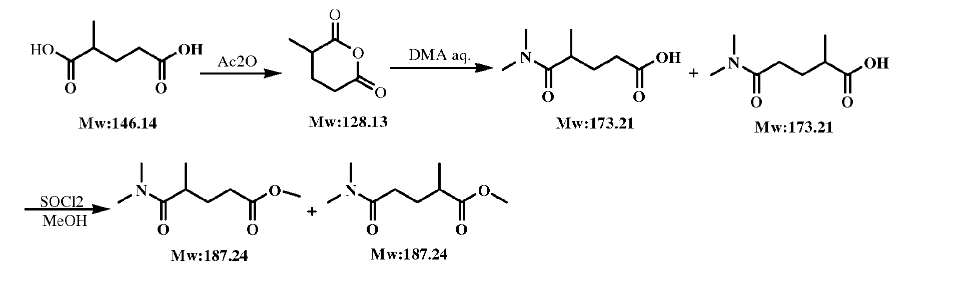

### Etape 1

### Matières premières

| Anhydride cyclique | DMA aq. |
|---|---|
| 264g; 2,06mol; 1eq. Mw: 128,13; | 1000ml; 6,84mol; 3.eq. Mw: 45,08; 33% dans l'eau |
| GC (surface) 99% | |

L'anhydride est obtenu par la Procédure F.
L'amide acide est obtenu selon la procédure D.
Produit brut = 368g (huile rouge) (isomères 29/71)

### Etape 2

### Matières premières

| amide-acide | Méthanol | Chlorure de thionyle |
|---|---|---|
| 236g; 1,36mol; 1 Eq | 2360ml; Mw: 32,04 | 236ml; 3,25mol; 2,4eq; Mw: |
| Mw: 173,21 | | 118,97; 99% |

Le méthyle ester diméthyle amide est obtenu selon la Procédure E.
Produit brut = 300g
Produit final = 171 g, Rendement = 68%
Analyse CG (surface) > 99% (isomères 31/69)

### Exemple 1.5 - Préparation de Isoamyl-OOC-A_{MG-}CONMe₂

La voie de synthèse est la suivante (seules les espèces majoritaires sont représentées):

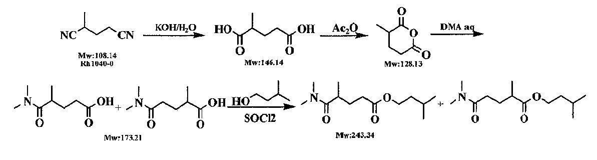

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite les étapes suivantes:

### Etape 1

### Matières premières

| Anhydride cyclique | DMA aq. |
|---|---|
| 780g; 6,09mol; 1 Eq | 2500mL; 16,31mo1; 2,6.eq. |
| Mw: 128,13; GC (surface) >99% | Mw: 45,08; 33% dans l'eau |

L'amide acide est obtenu selon la procédure D.
Produit brut = 1120g (liquide jaune) (isomères 29/71)

### Etape 2

### Matières premières

| amide-acide | 3-méthyle-1-butanol | Chlorure de thionyle |
|---|---|---|
| 1510g; 8,2mol; 1Eq | 2869ml; 25,7mol; 3.2Eq | 1446mL; 19,69mol; 2,5eq |
| Mw: 173,21 | Mw: 88,15 | Mw: 118,97; 99% |

L'ester diméthyle amide est obtenu selon la Procédure E.
Produit brut = 300g
Produit final = 1242g (Point d'ébullition - 132° /70Pa)
Analyse CG (surface) > 96% (isomères 31/69)
On donne ci-dessous le détail de l'analyse CG.

| Composé | CG (surface) |
|---|---|
| 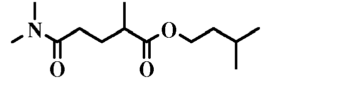 | 54,72% |
| 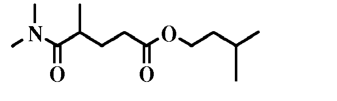 | 21,25% |
| 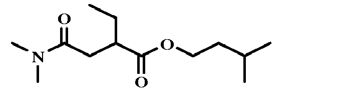 | 5,11% |
| 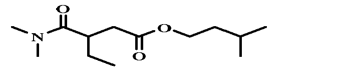 | 2,75 % |
| 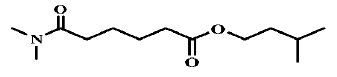 | 0,59% |
| Diester(s) | 14,97% |

### Exemple 1.6 - Préparation de cyclohexyl-OOC-A_{MG}-CONMe₂

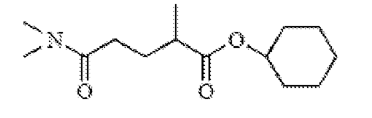

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite les étapes suivantes:

### Etape 1

### Matières premières

| Anhydride cyclique | DMA aq. |
|---|---|
| 331 g; 2,58mol; 1 Eq | 1030mL; 6,72mol; 2,6 eq. |
| Mw: 128,13; GC (surface) 99% | Mw: 45,08; 33% dans l'eau |

L'amide acide est obtenu selon la procédure D.
Produit brut = 440g (liquide jaune) (isomères 29/71), 94.7% CG

### Etape 2

### Matières premières

| Amide-acide | Cyclohexanol | Chlorure de thionyle |
|---|---|---|
| 400g; 2,31 mol; 1Eq | 289g; 1,25 mol Eq | 302g; 2,54mol; 1,1eq |
| Mw: 173,21 | Mw: 100 | Mw: 118,97; 99% |

L'ester diméthyle amide est obtenu selon la Procédure E sauf que l'amide acide est dissout dans du dichlorométhane avant la réaction.
Produit final = 430g (Point d'ébullition - 140-144° /40Pa), % Rendement = 73%
Analyse CG (surface) > 97%
On donne ci-dessous le détail de l'analyse CG

| **Composé** | **CG (surface)** |
|---|---|
| 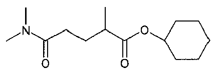 | 52,05% |
| 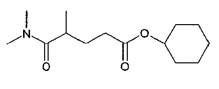 | 33,71% |
| 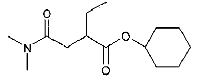 | 7,47% |
| 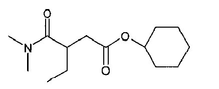 | 2,95% |
| 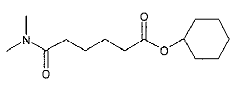 | 0,83% |
| 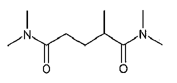 | 1,41% |
| 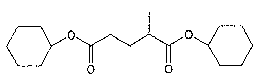 | 0,75% |

### Exemple 1.7 - Préparation de 2-ethylhexyl-OOC-A_{MG}_CONMe₂

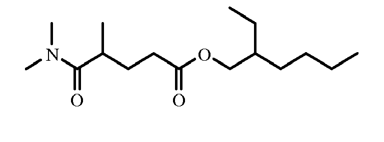

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite la procédure D.
On opère ensuite les étapes suivantes:

### Etape 1

### Matières premières

| Amide-acide | 2-Ethylhexanol | Chlorure de thionyle |
|---|---|---|
| 350g; 2,02mol; 1 Eq | 395mL; 2,53mol; 1.25Eq | 160mL; 2,22mol; 1, 1 eq |
| Mw: 173,21 | Mw: 130 | Mw: 118,97; 99% |

L'ester diméthyle amide est obtenu selon la Procédure E sauf que l'amide acide est dissout dans du dichlorométhane avant la réaction.
Produit final = 308g (Point d'ébullition - 148-150°/ 80Pa),
Rendement = 52%
Analyse CG (surface) > 96%
On donne ci-dessous le détail de l'analyse CG

| | |
|---|---|
| 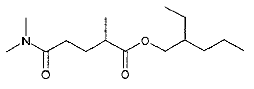 | 58,68% |
| 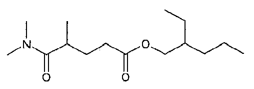 | 26,91% |
| 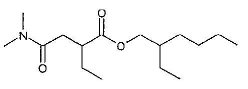 | 7,82% |
| 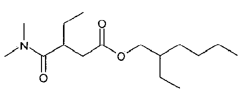 | 2,26% |
| 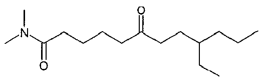 | 0,37% |
| Diester(s) | 3,26% |

### Exemple 1.8 - Préparation de Isoamyl-OOC-A_{MG-}CONEt₂

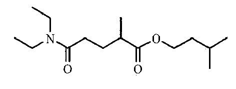

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite les étapes suivantes:

### Etape 1

### Matières premières

| Anhydride cyclique | DEA aq. |
|---|---|
| 420g; 3,28mol; 1 Eq | 880mL;8,53mo1; 2,6.eq. |
| Mw: 128,13; GC (surface) 99% | Mw: 73,1 |

L'amide acide est obtenu selon la procédure D.
Produit brut = 765g (liquide jaune), 90% par CG

### Etape 2

### Matières premières

| Amide-acide | 3-méthylbutan-1-ol | Chlorure de thionyle |
|---|---|---|
| 660g; 3,3mol; 1 Eq | 364g; 1.25 mol Eq | 426g; 3,6mol; 1,1 eq |
| Mw: 173,21 | Mw: 88,15 | Mw: 118,97; 99% |

L'ester diéthyl amide est obtenu selon la Procédure E sauf que l'amide acide est dissout dans du dichlorométhane avant la réaction.
Produit final = 460g (Point d'ébullition ∼ 150-158°/ 200Pa), % Rendement = 64% Analyse CG (surface) > 98%
On donne ci-dessous le détail de l'analyse CG

| **Composé** | **CG (surface)** |
|---|---|
| 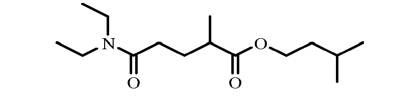 | 70,25% |
| 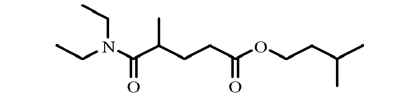 | 16,07% |
| 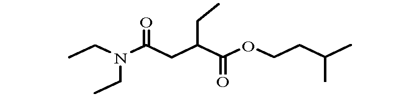 | 8,49% |
| 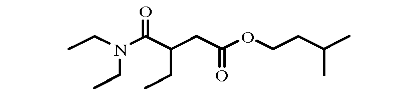 | 4,45% |
| 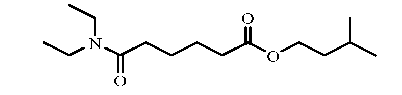 | 0,33% |
| Di-amide | 0,41% |
| Di-ester isomer | N.D |
| Total surface CG% | 100% |

### Exemple 1.9 - Préparation de Cyclohexyl-OOC-A_{MG}-CONEt₂

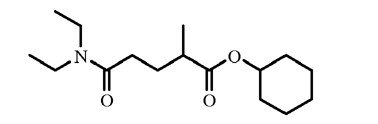

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite la procédure D.
On opère ensuite les étapes suivantes:

### Matières premières

| Amide-acide | Cyclohexanol | Chlorure de thionyle |
|---|---|---|
| 402g; 2,0mol; 1 Eq | 240g; 2,4mol; 1,2Eq | 160mL; 2,2mol; 1,1 eq |
| Mw: 173,21 | Mw: 100 | Mw: 118,97; 99% |

L'ester diéthyl amide est obtenu selon la Procédure E sauf que l'amide acide est dissout dans du dichlorométhane avant la réaction.
Produit final = 418g (Point d'ébullition - 142-146°/ 80Pa), % Rendement = 73.7% Analyse CG (surface) > 98%
On donne ci-dessous le détail de l'analyse CG

| **Composé** | **CG (surface)** |
|---|---|
| Divers | 0,53% |
| Diester(s) | 0,04% |
| 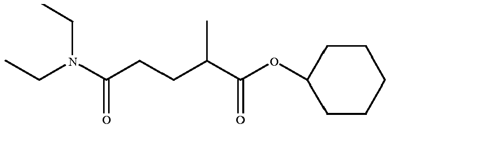 | 70,65% |
| 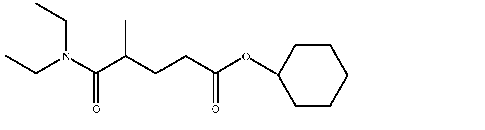 | 17,22% |
| 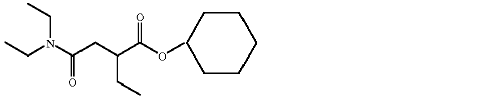 | 7,03% |
| 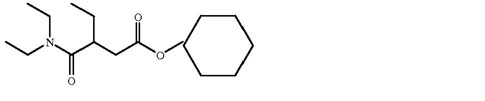 | 3,52% |
| 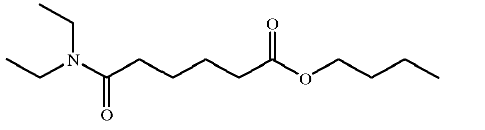 | 0,74% |
| Diamides | 0,26% |
| Total surface CG% | 100% |

### Exemple 1.10 - Préparation de n-buthyl-OOC-A_{MG}-CONEt₂

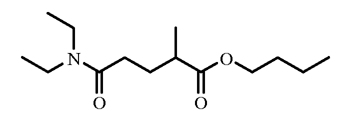

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite la procédure D.
On opère ensuite les étapes suivantes:

### Matières premières

| Amide-acide | n-butanol | Chlorure de thionyle |
|---|---|---|
| 500g, 2.335mol, 1 Eq | 270mL, 2.92mol, 1.25Eq | 187mL, 2.57mol, 1.1 eq |
| Mw: 173.21 | Mw: 74 | Mw: 118.97, 99% |

L'ester diéthyl amide est obtenu selon la Procédure E sauf que l'amide acide est dissout dans du dichlorométhane avant la réaction.
Produit final = 443g (Point d'ébullition ∼ 144°C / 80Pa), % Rendement = 72.8% Analyse CG (surface) > 98%
On donne ci-dessous le détail de l'analyse CG

| **Composé** | **CG (surface)** |
|---|---|
| Divers | 0,22% |
| Diester(s) | 0,36% |
| 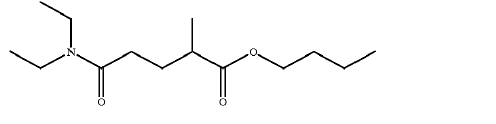 | 71,07% |
| 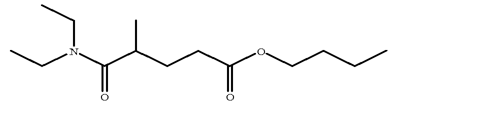 | 17,98% |
| 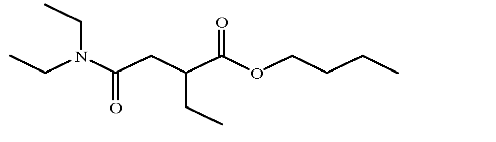 | 6,88% |
| 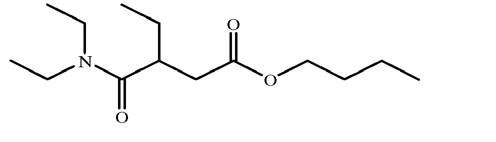 | 2,61% |
| 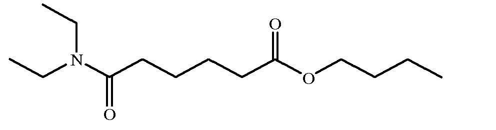 | 0,76% |
| Diamide(s) | 0,12% |
| Total GC area% | 100% |

### Exemple 1.11 - Préparation d'un mélange comprenant - MeOOC-A_{MG}-CONMe₂ (composé principal) et MeOOC-A_{ES}-CONMe₂

La formule du composé principal est

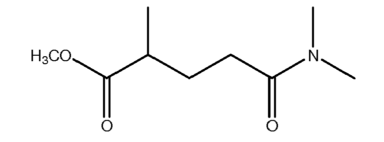

### Matières premières

| Methanol | Dimethylamine | Methylate de sodium dans methanol | Diesters methyliques | Acide sulfurique |
|---|---|---|---|---|
| 800 Kg | 1300 Kg | 240 Kg (60 Kg MeONa) | 4080 Kg | 55 Kg |
| | 28,89 Kmol | 1,11 Kmol | 23,45 Kmol | 0,55 Kmol |

On réalise une réaction de transamidification sur un mélange de diesters methyliques, comprenant du diméthyle 2-méthylglutarate (85% en poids), d'éthylsuccinate (12% en poids) et d'adipate (3% en poids).

A un mélange de méthanol anhydre et de diméthylamine gaz refroidi à 5 +/- 5°C, on additionne le méthylate de sodium en solution méthanolique, puis lentement, sur 4 heures le mélange de diesters méthyliques en maintenant la température à 10 +/- 5°C. La réaction est achevée en 8 heures à 15 +/- 5°C.

L'excès de diméthylamine est alors éliminé par distillation jusqu'à une température de 25 +/- 5°C et un vide de 200 mb en entrainant du méthanol. Le mélange condensé de diméthylamine en solution dans le méthanol est recyclé dans la charge suivante.

Le méthylate de sodium catalytique est neutralisé par de l'acide sulfurique concentré ou par des résines échangeuses d'ions (résines sulfoniques de type Amberlist ou Amberlit). Le sulfate de sodium ou la résine est éliminé du milieu par filtration et rincé par du méthanol frais.

Le méthanol est ensuite éliminé par distillation sous vide (jusqu'à 120°C et 10 mb) entrainant les diesters méthyliques n'ayant pas réagi (représentant 1 % de rendement) ; le mélange de méthanol et de diesters méthyliques est recyclé dans la production des diesters méthyliques.

Le produit est alors distillé sous une température maximale dans le bouilleur de 140°C et un vide de 5 mb ; on en récupère 4050 Kg, représentant un rendement de 92,3%

Le culot de distillation contient encore 280 Kg de produit (rendement de 6,3%) ; il est recyclé dans la distillation de l'opération suivante.

L'analyse type du produit distillé est la suivante :
Apparence : liquide limpide incolore à jaune clair.
Coloration : 100 APHA max
Analyse CG : Somme des isomères ester-amide : 96% min
   Somme des isomères diamide : 3 +/- 1%
   Somme des isomères diester n'ayant pas réagi : 0,5% max
   Methanol : 500 ppm max
Teneur en eau : 100 ppm max
Indice d'acide : 0,8 mg KOH / g de Produit max

### Exemple 1.12 - Préparation de t-butyl-OOC-A_{MG}-CONMe₂

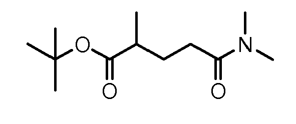

### La voie de synthèse est la suivante (seules les espèces majoritaires sont représentées):

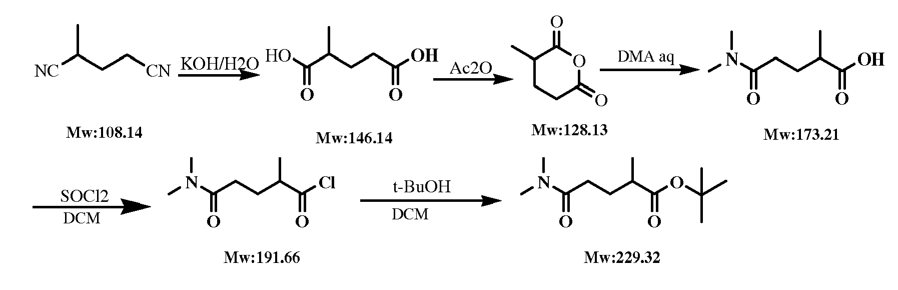

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite les étapes suivantes:

### Etape 1

### Matières premières

| Anhydride cyclique | DMA aq. |
|---|---|
| 370g; 2,89mol;1eq. Mw: 128,13; | 1152ml; 7,51 mol; 2,6eq. Mw: 45,08; 33% |
| GC (surface) 99% | dans l'eau |

L'amide acide est obtenu selon la procédure D.
Produit brut = 550g (liquide jaune pale)

### Etape 2

### Matières premières

| amide-acide | Dichlorométhane | Chlorure de thionyle |
|---|---|---|
| 592g; 3,42mol; 1 Eq | 1000mL | 274ml; 3,76mol; 1,1 eq; Mw: |
| Mw: 173,21 | | 118,97; 99% |

L'amide-acide est mélangé avec le dichlorométhane puis refroidi a environ 4°C. Le chlorure de thionyle est chargé lentement en environ 1.5h tout en contrôlant la température en dessous de 25°C. Le mélange réactionnel est agité à température ambiante pendant 10h. Les espèces volatiles sont retires pour obtenir le produit brut.
Produit brut = 687g (liquide foncé).

### Etape 3

### Matières premières

| Produit brut Etape 2 | Dichlorométhane | Tert-butanol |
|---|---|---|
| 305g; 1,52mol; 1 Eq | 300mL+300mL | 72ml; 0.77mol; 2eq; Mw: 74; 99% |
| Mw: 191,66 | | |

Le dichlorométhane est mélangé avec le tert-butanol et refroidi a 4°C. Le produit brut de l'étape 2, dilué dans le dichlorométhane, est alors ajouté lentement durant environ 1.5h tout en contrôlant la température en dessous de 10°C. Les volatiles sont retirés au rotoévaporateur. Le brut de réaction est traité avec 1500g de sodium hydrogénocarbonate puis filtré. Le gâteau est lavé avec 1500mL de dichlorométhane et le filtrat est séché sur sodium sulfate. Apres évaporation du solvant le produit brut est obtenu. Ce dernier est purifié par distillation (120°C/250Pa).
Produit brut = 301 g
Produit final = 150g
Analyse CG (surface) > 98% (isomères 8/92)

### Exemple 1.13 - Préparation de Et-butyl-OOC-A_{MG}-CONMe₂

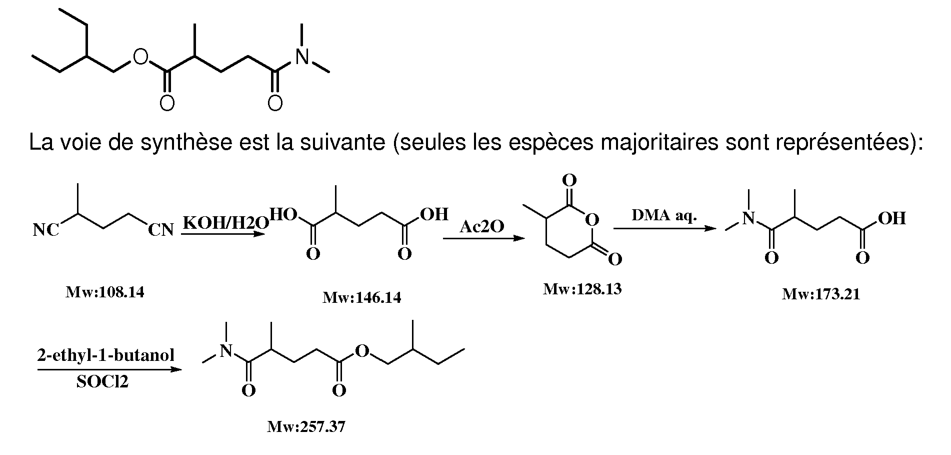

On hydrolyse un mélange appelé "MGN" brut comprenant du 2-méthylglutaroinitrile (2-MGN) en majorité, de l'éthylsuccinonitrile (ESN) et de l'adiponitrile (ADN) de manière à obtenir un mélange appelé MGA: Mélange comprenant de l'acide 2-methyl-glutarique (86% en moles), de l'acide Ethyl-succinique (11% en moles) et de l'acide adipique (3% en moles)
On opère une transformation en anhydride selon la procédure F.
On opère ensuite les étapes suivantes:

### Etape 1

### Matières premières

| Anhydride cyclique | DMA aq. |
|---|---|
| 397g; 3,1 mol; 1 eq. Mw: 128,13; GC (surface) 99% | 1237ml; 8,06mol; 2,6eq. Mw: 45,08; 33% dans l'eau |

L'amide acide est obtenu selon la procédure D.
Produit brut = 599g (liquide jaune pale)

### Etape 2

### Matières premières

| amide-acide | 2-ethyl butanol | dichlorométhane | Chlorure de thionyle |
|---|---|---|---|
| 599g; 3,46mol; 1 Eq | 721g; 6,92 mol ; | 550mL | 253ml; 3,49mol; 1,01 eq; |
| Mw: 173,21 | Mw: 32,04 | | Mw: 118,97; 99% |

Le méthyle ester diméthyle amide est obtenu selon la Procédure E.
Produit brut = 772g
Produit final = 420g, Rendement = 51,5%
Analyse CG (surface) > 98% (isomères 8/92)

### Exemples 2.1 et suivants - Utilisations comme solvants - Formulations phytosanitaires

Par mélange des ingrédients, on prépare des formulations de divers actifs phytosanitaires, de type concentré émulsionnable (EC).

Les formulations comprennent:
- l'actif, en quantité en poids (de matière active) indiquée dans le tableau ci-dessous,
- 10% en poids de tensioactif Alkamuls® RC, commercialisé par Rhodia
- et, comme solvant, le reste de composé ou composition de matière des exemples.

L'exemple 2.1.1 est un exemple comparatif où est utilisé comme solvant le produit Rhodiasolv® ADMA10, Rhodia (zone Asie Pacifique): Solvant alkyldiméthylamide.

On effectue les tests suivants:
- Observation visuelle à 25°C - On note l'aspect de la formulation et on repère éventuellement la présence de cristaux
- Observation visuelle à 0°C - On place la formulation pendant 7 jours à 0°C et on note l'aspect de la formulation et on repère éventuellement la présence de cristaux (test CIPAC MT39)
- Observation visuelle à 0°C avec nucléation: On introduit un cristal de la matière active dans la formulation ayant passé 7 jours à 0 °C pour nucléation, et on place à nouveau la formulation pendant 7 jours à 0 °C. On note l'aspect de la formulation et on repère éventuellement la présence de cristaux.

| *Exemple* | *Solvant* | *Actif* | *Apparence à 25 °C* | *Apparence à 0°C* | *Apparence à 0°C avec nucléation* |
|---|---|---|---|---|---|
| 2.1.1C | Rhodiasolv® ADMA 10 | Oxyfluorfen - 22% | Limpide | Limpide | Cristaux |
| 2.1.2C | Rhodiasolv® ADMA 10 | Propuxur - 20% | Limpide | Limpide | Cristaux |
| 2.1.3C | Rhodiasolv® ADMA 10 | Dimethoate - 40% | Trouble | Trouble | Cristaux |
| 2.1.4C | Rhodiasolv® ADMA 10 | Alachlor - 48% | Limpide | Cristaux | Cristaux |
| 2.1.10C | Rhodiasolv® ADMA 10 | Difenconazole - 25% | Limpide | Limpide | Limpide |
| 2.1.11C | Rhodiasolv® ADMA 10 | Triadimenol - 23% | Limpide | Limpide | Cristaux |
| 2.2.2 | Exemple 1.1 | Propuxur - 20% | Limpide | Limpide | Limpide |
| 2.2.3 | Exemple 1.1 | Dimethoate - 40% | Limpide | Limpide | Limpide |
| 2.2.4 | Exemple 1.1 | Alachlor - 48% | Limpide | Limpide | Cristaux |
| 2.2.6 | Exemple 1.1 | Fastac - 10% | Limpide | Limpide | Limpide |
| 2.2.7 | Exemple 1.1 | Phenmedipham - 16% | Limpide | Limpide | Limpide |
| 2.2.8 | Exemple 1.1 | Propanil - 36% | Limpide | Limpide | Limpide |
| 2.2.9 | Exemple 1.1 | Tebuconazole - 25% | Limpide | Limpide | Limpide |
| 2.2.10 | Exemple 1.1 | Difenconazole - 25% | Limpide | Limpide | Limpide |
| 2.3.2 | Exemple 1.2 | Propuxur - 20% | Limpide | Limpide | Limpide |
| 2.3.3 | Exemple 1.2 | Propuxur - 20% | Limpide | Limpide | Limpide |
| 2.3.5 | Exemple 1.2 | Chlorpyrifos - 40% | Limpide | Limpide | Limpide |
| 2.3.6 | Exemple 1.2 | Fastac - 10% | Limpide | Limpide | Limpide |
| 2.3.7 | Exemple 1.2 | Phenmedipham - 16% | Limpide | Limpide | Limpide |
| 2.3.9 | Exemple 1.2 | Tebuconazole - 25% | Limpide | Limpide | Limpide |
| 2.3.10 | Exemple 1.2 | Difenconazole - 25% | Limpide | Limpide | Limpide |
| 2.4.1 | Exemple 1.3 | Oxyfluorfen - 22% | Limpide | Limpide | Limpide |
| 2.4.2 | Exemple 1.3 | Propuxur - 20% | Limpide | Limpide | Limpide |
| 2.4.4 | Exemple 1.3 | Alachlor - 48% | Limpide | Limpide | Cristaux |
| 2.4.5 | Exemple 1.3 | Chlorpyrifos - 40% | Limpide | Limpide | Limpide |
| 2.4.6 | Exemple 1.3 | Fastac - 10% | Limpide | Limpide | Limpide |
| 2.4.7 | Exemple 1.3 | Phenmedipham - 16% | Limpide | Limpide | Limpide |
| 2.4.8 | Exemple 1.3 | Propanil - 36% | Limpide | Limpide | Limpide |
| 2.4.9 | Exemple 1.3 | Tebuconazole - 25% | Limpide | Limpide | Limpide |
| 2.5.4 | Exemple 1.5 | Alachlor - 48% | Limpide | Limpide | Cristaux |
| 2.5.5 | Exemple 1.5 | Chlorpyrifos - 40% | Limpide | Limpide | Limpide |
| 2.5.6 | Exemple 1.5 | Fastac - 10% | Limpide | Limpide | Limpide |
| 2.5.7 | Exemple 1.5 | Phenmedipham - 16% | Limpide | Limpide | Limpide |
| 2.5.8 | Exemple 1.5 | Propanil - 36% | Limpide | Limpide | Limpide |
| 2.5.9 | Exemple 1.5 | Tebuconazole - 25% | Limpide | Limpide | Limpide |
| 2.6.4 | Exemple 1.6 | Alachlor - 48% | Limpide | Limpide | Cristaux |
| 2.6.8 | Exemple 1.6 | Propanil - 36% | Limpide | Limpide | Limpide |
| 2.6.11 | Exemple 1.6 | Triadimenol - 23% | Limpide | Limpide | Limpide |
| 2.7.4 | Exemple 1.7 | Alachlor - 48% | Limpide | Limpide | Cristaux |
| 2.7.8 | Exemple 1.7 | Propanil - 36% | Limpide | Limpide | Limpide |
| 2.8.4 | Exemple 1.8 | Alachlor - 48% | Limpide | Limpide | Cristaux |
| 2.8.8 | Exemple 1.8 | Propanil - 36% | Limpide | Limpide | Limpide |
| 2.9.4 | Exemple 1.9 | Alachlor - 48% | Limpide | Limpide | Cristaux |
| 2.9.8 | Exemple 1.9 | Propanil - 36% | Limpide | Limpide | Limpide |
| 2.9.9 | Exemple 1.9 | Tebuconazole - 25% | Limpide | Limpide | Limpide |
| 2.10.4 | Exemple 1.10 | Alachlor - 48% | Limpide | Limpide | Cristaux |
| 2.10.8 | Exemple 1.10 | Propanil - 36% | Limpide | Limpide | Limpide |
| 2.10.10 | Exemple 1.10 | Difenconazole - 25% | Limpide | Limpide | Limpide |
| 2.11.1 | Exemple 1.11 | Oxyfluorfen - 22% | Limpide | Limpide | Limpide |
| 2.11.3 | Exemple 1.11 | Dimethoate - 40% | Limpide | Limpide | Cristaux |
| 2.11.4 | Exemple 1.11 | Propuxur - 20% | Limpide | Limpide | Limpide |
| 2.11.5 | Exemple 1.11 | Chlorpyrifos - 40% | Limpide | Limpide | Limpide |
| 2.11.7 | Exemple 1.11 | Phenmedipham - 16% | Limpide | Limpide | Limpide |
| 2.11.8 | Exemple 1.11 | Propanil - 36% | Limpide | Limpide | Limpide |
| 2.11.9 | Exemple 1.11 | Tebuconazole - 25% | Limpide | Limpide | Limpide |
| 2.11.10 | Exemple 1.11 | Difenconazole - 25% | Limpide | Limpide | Limpide |
| 2.12.4 | Exemple 1.12 | Alachlor - 48% | Limpide | Limpide | Cristaux |
| 2.12.5 | Exemple 1.12 | Chlorpyrifos - 40% | Limpide | Limpide | Limpide |
| 2.12.7 | Exemple 1.12 | Phenmedipham - 16% | Limpide | Limpide | Limpide |
| 2.12.8 | Exemple 1.12 | Propanil - 36% | Limpide | Limpide | Limpide |
| 2.12.9 | Exemple 1.12 | Tebuconazole - 25% | Limpide | Limpide | Limpide |
| 2.12.11 | Exemple 1.12 | Triadimenol - 23% | Limpide | Limpide | Cristaux |
| 2.13.3 | Exemple 1.13 | Dimethoate - 40% | Limpide | Limpide | Cristaux |
| 2.13.4 | Exemple 1.13 | Alachlor - 48% | Limpide | Limpide | Cristaux |

## Revendications

1. Utilisation comme solvant ou agent de coalescence, d'un composé esteramide seul ou en mélange de formule (I) suivante :
R¹OOC-A-CONR²R³ (I)
où:
- R¹ est un groupe choisi parmi les groupes hydrocarbonés comprenant un nombre moyen d'atomes de carbone allant de 1 à 36, saturés ou insaturés, linéaires ou ramifiés, éventuellement cycliques, éventuellement aromatiques,
- R² et R³, identiques ou différents, sont des groupes choisis parmi les groupes hydrocarbonés comprenant un nombre moyen d'atomes de carbone allant de 1 à 36, saturés ou insaturés, linéaires ou ramifiés, éventuellement cycliques, éventuellement aromatiques, éventuellement substitués, R² et R³ pouvant éventuellement former ensemble un cycle, éventuellement substitué et/ou comprenant éventuellement un hétéroatome, et
- A est un groupe alkyle divalent linéaire comprenant un nombre moyen d'atomes de carbone allant de 2 à 12.

2. Utilisation selon la revendication 1, **caractérisée en ce que** le composé esteramide est utilisé dans une composition phytosanitaire, une composition de nettoyage, de dégraissage ou de décapage.

3. Utilisation selon l'une des revendications précédentes, **caractérisée en ce que** si R² et R³ sont des groupes éthyles, alors le composé esteramide est utilisé dans une composition phytosanitaire, une composition de nettoyage, de dégraissage ou de décapage.

4. Utilisation selon l'une des revendications précédentes, **caractérisée en ce que** R¹, R² et R ³ identiques ou différents, sont des groupes choisis parmi les groupes alkyle, aryle, alkaryle, arylalkyle, en C₁-C₁₂ ou le groupe phényle, R² et/ou R³ étant éventuellement substitués.

5. Utilisation selon l'une des revendications précédentes, **caractérisée en ce que** R¹ est choisi parmi les groupes méthyle, éthyle, propyle, isopropyle, n-butyle, isobutyle, n-pentyle, isopentyle, isoamyle, n-hexyle, cyclohexyle, 2-éthylbutyle, n-octyle, isooctyle, 2-ethylhexyle, et tridécyle.

6. Utilisation selon l'une des revendications précédentes, **caractérisée en ce que** R² et R³, identiques ou différents, sont choisis parmi les groupes méthyle, éthyle, propyle (n-propyl), isopropyle, n-butyle, isobutyle, n-pentyle, amyle, isoamyle, hexyle, cyclohexyle, hydroxyéthyle, morpholine, pipérazine et pipéridine.

7. Utilisation selon l'une des revendications précédentes, **caractérisée en ce que** A est un groupe alkyle linéaire divalent choisi parmi les groupes de formules suivantes :
-CH₂-CH₂- (éthylène); -CH₂-CH₂-CH₂- (n-propylène); -CH₂-CH₂-CH₂-CH₂- (n-butylène), et leurs mélanges.

8. Utilisation selon l'une des revendications précédentes, **caractérisée en ce que** le composé esteramide est choisi parmi les composés suivants :
- MeOOC-CH₂-CH₂-CONMe₂
- MeOOC-CH₂-CH₂-CH₂-CONMe₂

9. Utilisation selon l'une des revendications précédentes, **caractérisée en ce que** l'esteramide est différent des composés suivants:
- MeOOC-CH₂-CH₂-CH₂-CONMe₂
- MeOOC-CH₂-CH₂-CONMe₂
si ces derniers ne sont pas utilisés en mélange avec d'autres composés répondant à la formule (1).

10. Utilisation selon l'une des revendications précédentes, **caractérisée en ce que** le composé esteramide est différent des composés ou mélanges suivants :
- MeOOC-CH₂-CH₂-CH₂-CONMe₂
- MeOOC-CH₂-CH₂-CONMe₂
si ces derniers, individuellement, ne sont pas utilisés en mélange avec d'autres composés répondant à la formule (I).

11. Utilisation selon l'une des revendications précédentes, **caractérisée en ce que** l'esteramide a un point de fusion inférieur à ou égal à 20°C.

12. Utilisation selon l'une des revendications précédentes, dans une formulation phytosanitaire, dans une formulation du nettoyage, dans une formulation de décapage, dans une formulation de dégraissage, dans une formulation de lubrifiants, dans une formulation de revêtement, dans une formulation de pigments ou encre.

13. Procédé de préparation d'un composé esteramide tel que défini dans l'une des revendications 1 à 11, comprenant une étape de réaction d'un anhydride de formule (I'), avec un alcool de formule R¹-OH et/ou une amine de formule HNR²R³

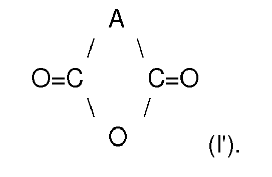

14. Procédé selon la revendication 13, **caractérisé en ce que** l'anhydride est préparé lors d'une étape a) préalable de cyclisation d'un diacide de formule HOOC-A-COOH, de préférence par réaction du diacide avec de l'anhydride acétique.

15. Procédé selon l'une des revendications 13 ou 14, **caractérisé en ce qu'**on met en oeuvre l'une des séquences réactionnelles 1) ou 2) suivantes :
Séquence 1):
Etape 1b) on fait réagir l'anhydride de formule (1') avec un alcool de formule R¹⁻ OH, de manière à obtenir un composé ester-acide de formule (1")
R¹-OOC-A-COOH (l")
,
Etape 1c) on transforme le composé de formule (1") en composé de formule (1) à l'aide d'une amine de formule HNR²R³,
Séquence 2):
Etape 2b) on fait réagir l'anhydride de formule (I') avec une amine de formule HNR²R³ de manière à obtenir un composé amide-acide de formule (II")
HOOC-A-CONR²R³ (II")
,
Etape 2c) on transforme le composé de formule (II") en composé de formule (I) à l'aide d'un alcool de formule R¹-OH.

16. Procédé de préparation d'un composé esteramide tel que défini dans l'une des revendications 1 à 11, comprenant une étape de réaction d'un diester de formule R¹OOC-A-COOR¹ avec une amine de formule HNR²R³, puis éventuellement une étape de réaction avec un alcool de formule R^{1'}-OH, où R^{1'} est un groupe choisi parmi les groupes R¹, mais différent du groupe R¹ du diester.

17. Composé esteramide, seul ou en mélange, de formule (I) suivante :
R¹OOC-A-CONR²R³ (I)
où:
- R¹ est un groupe choisi parmi les groupes hydrocarbonés comprenant un nombre moyen d'atome de carbone allant de 1 à 36, saturés ou insaturés, linéaires ou ramifiés, éventuellement cycliques, éventuellement aromatiques,
- R² et R ³ identiques ou différents, sont des groupes choisis parmi les groupes hydrocarbonés comprenant un nombre moyen d'atome de carbone allant de 1 à 36, saturés ou insaturés, linéaires ou ramifiés, éventuellement cycliques, éventuellement aromatiques, éventuellement substitués, R² et R³ pouvant éventuellement former ensemble un cycle, éventuellement substitué et/ou comprenant éventuellement un hétéroatome, et
- A est un groupe alkyle divalent linéaire comprenant un nombre moyen d'atomes de carbone allant de 2 à 12, à l'exception des composés ou mélanges suivants:
- MeOOC-CH₂-CH₂-CH₂-CONMe₂
- MeOOC-CH₂-CH₂-CONMe₂
si ces derniers composés ou mélanges exclu, individuellement, ne sont pas utilisés en mélange avec d'autres composés répondant à la formule (I).

18. Composé selon la revendication 17, **caractérisé en ce que** R¹, R² et R³ identiques ou différents, sont des groupes choisis parmi les groupes alkyle, aryle, alkaryle, arylalkyle en C₁-C₁₂ ou le groupe phényle, R² et/ou R³ étant éventuellement substitués.

19. Composé selon la revendication 17 ou 18, **caractérisé en ce que** R¹ est choisi parmi les groupes méthyle, éthyle, propyle, isopropyle, n-butyle, isobutyle, n-pentyle, isopentyle, isoamyle, n-hexyle, cyclohexyle, 2-éthylbutyl, n-octyle, isooctyle, 2-ethylhexyle et tridécyle.

20. Composé selon l'une des revendications 17 à 19, **caractérisé en ce que** R² et R³, identiques ou différents, sont choisis parmi les groupes méthyle, éthyle, propyle (n-propyl), isopropyle, n-butyle, isobutyle, n-pentyle, amyle, isoamyle, hexyle, cyclohexyle, hydroxyéthyle, morpholine, pipérazine et pipéridine.

21. Composé selon l'une des revendications 17 à 20, **caractérisé en ce que** A est un groupe alkyle linéaire divalent choisi parmi les groupes de formules suivantes : -CH₂-CH₂- (éthylène); -CH₂-CH₂-CH₂- (n-propylène); -CH₂-CH₂-CH₂-CH₂- (n-butylène), et leurs mélanges.

22. Composé selon l'une des revendications 17 à 21, **caractérisé en ce que** le composé esteramide est choisi parmi les composés suivants :
- MeOOC-CH₂-CH₂-CH₂-CONMe₂, en mélange avec MeOOC-CH₂CH₂-CH₂-CH₂-CONMe₂ et/ou avec MeOOC-CH₂-CH₂-CONMe₂.
